(19) **Europäisches Patentamt**
**European Patent Office**
**Office européen des brevets**

(11) **EP 2 792 751 B1**

(12) **EUROPEAN PATENT SPECIFICATION**

(45) Date of publication and mention
of the grant of the patent:
**05.09.2018 Bulletin 2018/36**

(21) Application number: **11877603.8**

(22) Date of filing: **17.12.2011**

(51) Int Cl.:
***C12Q 1/68*** *(2018.01)*

(86) International application number:
**PCT/CN2011/084165**

(87) International publication number:
**WO 2013/086744 (20.06.2013 Gazette 2013/25)**

(54) **METHOD AND SYSTEM FOR DETERMINING WHETHER GENOME IS ABNORMAL**

VERFAHREN UND SYSTEM ZUR BESTIMMUNG DER ANOMALIE EINES GENOMS

PROCÉDÉ ET SYSTÈME POUR DÉTERMINER SI UN GÉNOME EST ANORMAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**22.10.2014 Bulletin 2014/43**

(73) Proprietor: **BGI Genomics Co., Ltd.**
**Yantian District**
**Shenzhen, Guangdong Province 518083 (CN)**

(72) Inventors:
• **QIU, Yong**
**Shenzhen**
**Guangdong 518083 (CN)**
• **LIU, Lifu**
**Shenzhen**
**Guangdong 518083 (CN)**
• **JIANG, Hui**
**Shenzhen**
**Guangdong 518083 (CN)**
• **CHEN, Fang**
**Shenzhen**
**Guangdong 518083 (CN)**
• **ZHANG, Chunlei**
**Shenzhen**
**Guangdong 518083 (CN)**
• **WANG, Jian**
**Shenzhen**
**Guangdong 518083 (CN)**
• **WANG, Jun**
**Shenzhen**
**Guangdong 518083 (CN)**

• **YANG, Huanming**
**Shenzhen**
**Guangdong 518083 (CN)**
• **ZHANG, Xiuqing**
**Shenzhen**
**Guangdong 518083 (CN)**

(74) Representative: **Rössler, Matthias et al**
**Kahlhöfer Rößler Kreuels**
**Patentanwälte PartG mbB**
**(Karo IP Professionals)**
**Platz der Ideen 2**
**40476 Düsseldorf (DE)**

(56) References cited:
**WO-A2-2007/147079     CN-A- 101 305 087
CN-A- 101 675 169**

• **HAHN S ET AL: "Determination of fetal
chromosome aberrations from fetal DNA in
maternal blood: has the challenge finally been
met?", EXPERT REVIEWS IN MOLECULAR
MEDICINE, CAMBRIDGE UNIVERSITY PRESS,
CAMBRIDGE, vol. 13, 4 May 2011 (2011-05-04),
pages e16-1, XP002685359, ISSN: 1462-3994,
DOI: 10.1017/S1462399411001852**
• **CHIU ROSSA W K ET AL: "Noninvasive prenatal
diagnosis of fetal chromosomal aneuploidy by
massively parallel genomic sequencing of DNA
in maternal plasma", PROCEEDINGS OF THE
NATIONAL ACADEMY OF SCIENCES, NATIONAL
ACADEMY OF SCIENCES, US, vol. 105, no. 51, 23
December 2008 (2008-12-23), pages 20458-20463,
XP002620454, ISSN: 0027-8424, DOI:
10.1073/PNAS.0810641105 [retrieved on
2008-12-10]**

- BÜSCH J ET AL: "Enrichment of fetal cells from maternal blood by high gradient magnetic cell sorting (double MACS) for PCR-based genetic analysis", PRENATAL DIAGNOSIS, CHICHESTER, SUSSEX, GB, vol. 14, no. 12, 1 December 1994 (1994-12-01), pages 1129-1140, XP002513168, ISSN: 0197-3851, DOI: 10.1002/PD.1970141206
- GAENSHIRT-AHLERT D ET AL: "DETECTION OF FETAL TRISOMIES 21 AND 18 FROM MATERNAL BLOOD USING TRIPLE GRADIENT AND MAGNETIC CELL SORTING", AMERICAN JOURNAL OF REPRODUCTIVE IMMUNOLOGY, WILEY-BLACKWELL PUBLISHING, INC, US, vol. 30, no. 2/03, 1 September 1993 (1993-09-01), pages 194-201, XP001011403, ISSN: 1046-7408
- Eric Z. Chen ET AL: "Noninvasive Prenatal Diagnosis of Fetal Trisomy 18 and Trisomy 13 by Maternal Plasma DNA Sequencing", PLoS ONE, vol. 6, no. 7, 6 July 2011 (2011-07-06), page e21791, XP55024137, DOI: 10.1371/journal.pone.0021791

**Description**

**Technical Field**

The present invention relates to the biomedical field. Specifically, it relates to a method and system for determining whether a genomic abnormality exists, and more specifically, the present invention relates to a method for determining the genomic sequence of fetal nucleated red blood cells, a method for determining whether a genomic abnormality exists, and a system for determining whether a genomic abnormality exists.

**Background Art**

Prenatal diagnosis, also known as pre-birth diagnosis, refers to making a high-accuracy diagnosis on whether a fetus before birth suffers from certain genetic diseases or congenital malformations by combining genetic detection and imaging examination results. Currently used methods for prenatal diagnosis are mainly classified into invasive diagnosis and non-invasive diagnosis according to the difference in sampling methods. The invasive diagnoses mainly include amniocentesis (amniotic fluid test), chorionic centesis, cord blood sampling, fetoscopy, embryo biopsy, etc. Currently, amniocentesis and chorionic centesis are relatively commonly applied. For the invasive diagnoses, because fetal cells or tissues can be sampled directly, an accurate and reliable result can be obtained after genetic detection. However, due to the invasive sampling process, potential harm can be brought to the pregnant woman and the fetus, and in a serious case, even fetal abortion or intrauterine infection can result. According to statistics, amniocentesis and chorionic centesis can lead to an abortion rate of about 1% and of 1%-2%, respectively.

Eric Z. Chen et al discloses a massively parallel sequencing of DNA molecules in the plasma of pregnant women has shown to allow accurate and noninvasive prenatal detection of fetal trisomy 21. However, whether he sequencing approach is as accurate for the noninvasive prenatal diagnosis of trisomy 13 and 18 is unclear due to the lack of data from a large sample set (Eric Z. Chen et al: "Noninvasive Prenatal Diagnosis of Fetal Trisomy 18 and Trisomy 13 by Maternal Plasma DNA Sequencing", PloS ONE, vol. 6, no. 7, 6 July 2011, page e21791).

Currently, there is a need to improve the methods for prenatal diagnosis.

**Summary of the Invention**

The present invention aims to solve at least one of the technical problems existing in the prior art. Therefore, the present invention provides a method and system capable of effectively determining whether a genomic abnormality exists.

According to an aspect of the present invention, the present invention provides a method for determining whether a genomic abnormality exists. According to embodiments of the present invention, the method for determining whether a genomic abnormality exists comprises the steps as defined in claim 1. The inventors found that it can be effectively determined whether a genomic abnormality exists in fetal nucleated red blood cells separated from a sample from a pregnant woman using a method according to embodiments of the present invention. The method can be for a non-medical purpose.

According to a second aspect of the present invention, the present invention provides a system for determining whether a genomic abnormality exists. According to embodiments of the present invention, the system is defined as in claim 9. The method for determining whether a genomic abnormality exists according to the embodiments of the present invention can be effectively implemented using the system for determining chromosomal aneuploidy in nucleated red blood cells, and thus, it can be effectively determined whether a genomic abnormality exists in nucleated red blood cells.

According to another aspect of the present invention, the present invention provides a method for determining the genomic sequence of fetal nucleated red blood cells. According to the embodiments of the present invention, the method comprises the steps of: separating fetal nucleated red blood cells from a sample from a pregnant woman; and sequencing at least a part of the genome of said nucleated red blood cells, so as to obtain a sequencing result. The information from the genomic sequence of nucleated red blood cells can be effectively determined using this method, and the information from the sequence of the fetal genome can thereby be determined.

The additional aspects and advantages of the present invention will partly be given in the following description, and partly become apparent from the following description, or be understood through the practice of the present invention.

**Description of the Drawings**

The above-mentioned and/or additional aspects and advantages of the present invention will become apparent and easy to understand from the description of the embodiments in conjunction with the following drawings, wherein:

Figure 1 shows a schematic flow diagram of a method for determining whether a genomic abnormality exists in nucleated cells according to an embodiment of the present invention.

Figure 2 shows a schematic flow diagram of a method for determining whether a genomic abnormality exists in nucleated cells according to another embodiment of the present invention.

Figure 3 shows a schematic diagram of a system used for determining whether a genomic abnormality exists in nucleated cells according to an embodiment of the present invention.

Figure 4 shows a schematic diagram of a nucleated red blood cell separation device according to an embodiment of the present invention.

Figure 5 shows a schematic diagram of a system used for determining whether a genomic abnormality exists in nucleated cells according to yet another embodiment of the present invention

Figure 6 shows a schematic diagram for a whole-genome sequencing library preparation device according to an embodiment of the present invention.

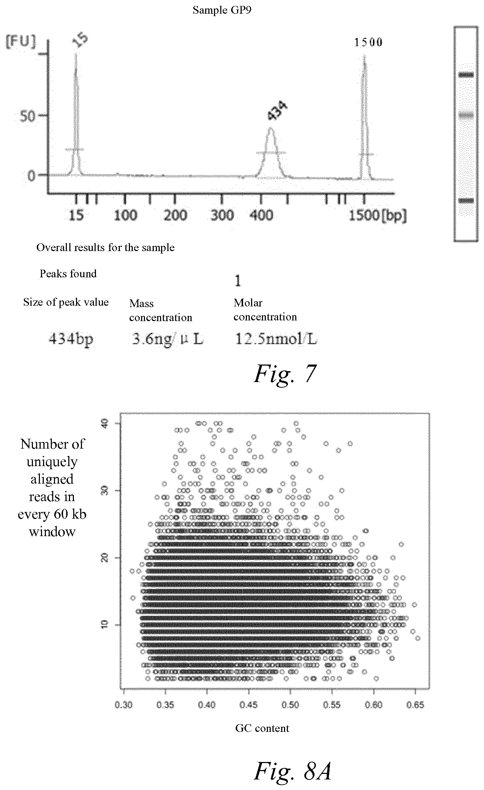

Figure 7 shows a detection result of a constructed DNA library analyzed by Agilent®Bioanalyzer 2100 according to an embodiment of the present invention. Briefly, an amplification product of the whole genome of isolated positive cells (nucleated red blood cells) was sheared by an ultrasonic wave, DNA fragments in the sheared main band were of about 350 bp, the lengths of the fragments after ligation to an adapter were increased by about 120 bp, and the fragments of 430-450 bp were recovered by excising from the gel. It can be seen from Figure 7 that the range of fragments of the four libraries meets requirements, and the quality of the library meets sequencing requirements, where GP9 is a test sample and YH6 is a control sample (a normal human sample).

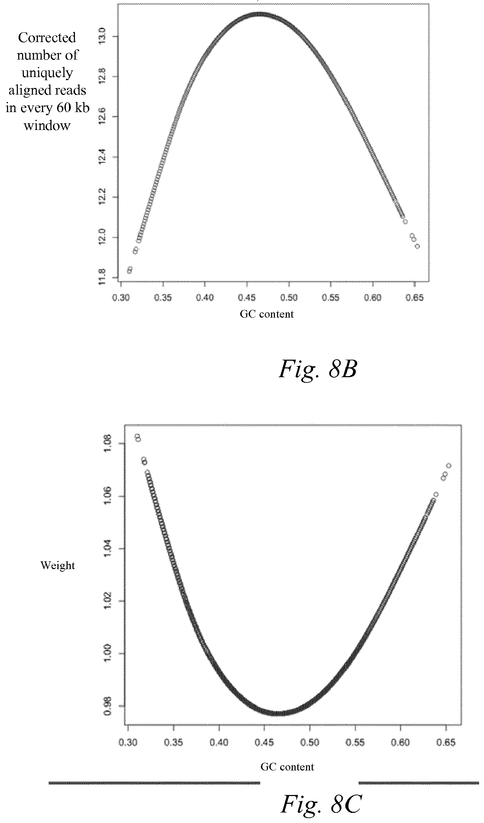

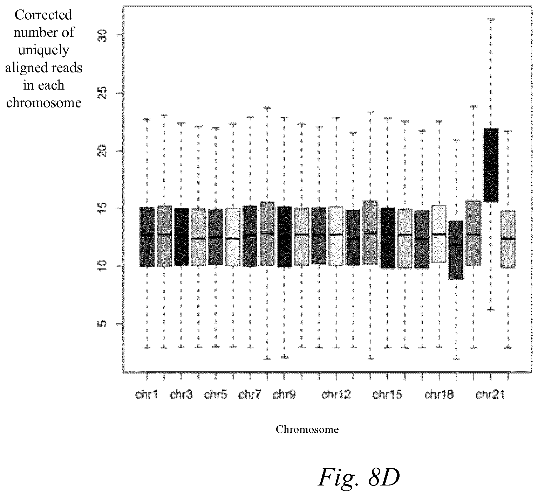

Figure 8 shows an analysis result of sequencing data according to an embodiment of the present invention, wherein, (A) shows the distribution of the GC value of each window and the number of uniquely aligned sequencing data for a sample to be tested; (B) shows the smooth spline fitted curve of the relationship between the GC content and the number of uniquely aligned sequencing data; (C) shows the distribution of the weighting coefficient corresponding to the correction of data of each window in the sample to be tested, where the window of each GC content corresponds to one UR value as a correction weight; and (D) shows a box plot showing sequencing data of each chromosome.

**Particular Embodiments**

Embodiments of the present invention are detailed below, and examples of said embodiments are shown in the drawings, where the same or similar labels represent the same or similar elements or elements with the same or similar functions from first to last. The following embodiments described by reference to the drawings are exemplary, are only used for explaining the present invention, and cannot be understood as a limitation on the present invention.

It needs to be noted that the terms "first" and "second" are only used for the purpose of describing, and cannot be understood as indicating or implying the relative importance or implicitly specifying the number of indicated technical features. Thus, features defined by "first" and "second" can explicitly or implicitly include one or more of the features. Furthermore, in the description of the present invention, unless otherwise noted, the meaning of "a plurality of' is two or more.

**1. Method for determining whether a genomic abnormality exists**

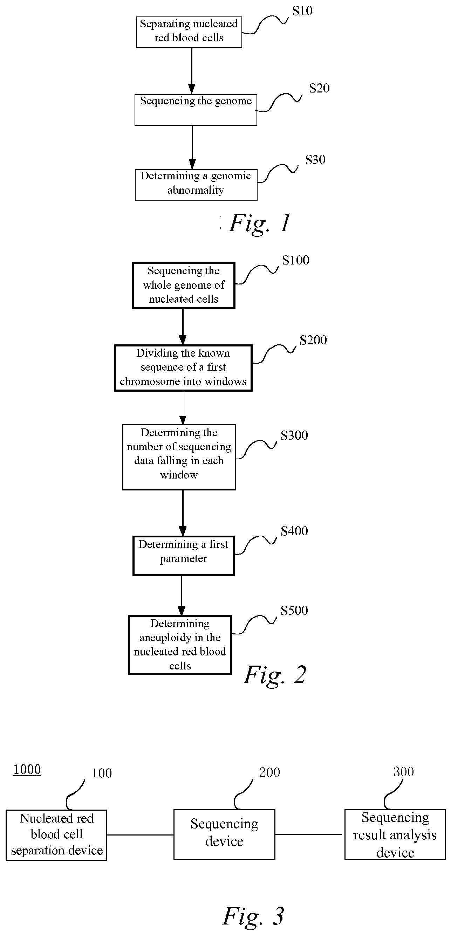

An aspect of the present invention relates to a method for determining whether an abnormality exists in a genome. Referring to Figure 1, according to embodiments of the present invention, the method comprises the steps of:

**S10: separating fetal nucleated red blood cells from a sample from a pregnant woman.**

In the present invention, the selection of separating fetal nucleated red blood cells from a sample from a pregnant woman is accomplished based on the following discovery by the inventors. Currently, studies on fetal genetic abnormalities are mainly based on separating free fetal DNA from a pregnant woman. However, besides free DNA of fetal origin, a large amount of DNA of maternal origin also exists in peripheral blood of the pregnant woman, and half of fetal genomic DNA is derived from the mother, making it relatively difficult to determine accurately the origin of DNA at present. In addition, free fetal DNA in maternal peripheral blood exists in an incomplete genome, which may thus greatly increase the false negative probability due to the loss of a template in the process of detecting a specific gene site. Therefore, the detection of fetal genetic abnormalities using free fetal DNA has its own defects. The inventors further discovered that besides free fetal nucleic acids, intact fetal cells also exist in the maternal peripheral blood. Fetal cells free in the peripheral blood of the pregnant woman mainly include: trophoblastic cells, white blood cells and fetal nucleated red blood cells. Among these cells, the inventors discovered that the trophoblast cells are prone to lead to misdiagnosis because of the existence of two forms of such cells, *i.e.,* a multinucleated form and a mononucleated form. The white blood cells will exist in the maternal blood persistently after the birth of the fetus, thus can interfere with the detection in the next pregnancy. The inventors discovered that the fetal nucleated red blood cells have a relatively short life cycle, will disappear within 90 days after the birth of the fetus, and will not interfere with the detection of the next pregnancy, and that antigens on the surface of the nucleated red blood cells are relatively stable, and can be easily recognized and separated. Thus, a fetal genomic abnormality can be effectively determined using the fetal nucleated red blood cells. The inventors found that using the fetal nucleated red blood cells in the peripheral blood of a pregnant woman to perform a non-invasive prenatal diagnosis in conjunction with high-throughput sequencing has achieved much more superior results than the currently available non-invasive prenatal diagnosis using the plasma of the pregnant woman.

According to embodiments of the present invention, the sample from a pregnant woman as the source of nucleated red blood cells is not particularly limited. According to some embodiments of the present invention, said sample from a pregnant woman is preferably peripheral blood of the pregnant woman. Thus, these samples can be acquired from the pregnant woman conveniently, and on the premise such that the fetal development is not affected. Fetal nucleated red blood cells can be obtained from the peripheral blood of the pregnant woman to perform whole-genome sequencing, thereby realizing non-invasive prenatal examination. In addition, the stage of pregnancy of a pregnant woman as the source of fetal nucleated red blood cells is not particularly limited. According to embodiments of the present invention, a sample from a pregnant woman with a gestational age below 20 weeks can be used for the isolation of fetal nucleated red blood cells. For example, a sample from a pregnant woman with a gestational age of 12-20 weeks can be adopted as a research object. Thus, fetal nucleated red blood cells can be more effectively isolated for further analysis. In addition, the inventors surprisingly discovered that with the use of the method of the present invention, even if only 1 fetal nucleated red blood cell is obtained, the analysis can still be performed effectively.

According to embodiments of the present invention, a method for separating nucleated red blood cells from a biological sample, e.g. peripheral blood, is not particularly limited. According to the particular embodiments of the present invention, separating said nucleated red blood cells from peripheral blood further comprises the steps of:
Firstly, gradient centrifugation is performed on said peripheral blood using a density gradient reagent, so as to obtain monocytes. According to embodiments of the present invention, the type of the density gradient reagent is not particularly limited, and according to the particular examples, polysucrose, e.g. Ficoll, can be utilized to form a density gradient. Preferably, said gradient centrifugation can be performed at 800×g for 30 minutes.

After the monocytes are obtained, nucleated red blood cells are enriched from the obtained monocytes using magnetic beads carrying an antibody, wherein the antibody carried on the magnetic beads specifically recognizes an antigen on the surface of the nucleated red blood cells. The nucleated red blood cells will bind to the magnetic beads through the antibody, and subsequently, the nucleated red blood cells can be obtained through magnetic screening.

According to an embodiment of the present invention, after said monocytes are obtained and before said nucleated red blood cells are enriched using the magnetic beads carrying anti-CD71 antibody, the process further comprises washing said monocytes using phosphate buffer saline (PBS buffer) containing 1% bovine serum albumin (BSA), so as to remove the residual density gradient reagent. Preferably, said PBS buffer contains potassium dihydrogen phosphate and disodium hydrogen phosphate, but is free of calcium ions and magnesium ions, to thereby significantly improve the efficiency of the enrichment of the nucleated red blood cells. According to the particular examples of the present invention, washing said monocytes using PBS buffer containing 1% BSA further comprises: mixing said monocytes and said PBS buffer containing 1% BSA, so as to obtain a suspension containing monocytes; and centrifuging said suspension containing monocytes, preferably, centrifuging the suspension at 200×g for 5 minutes, and discarding the supernatant, so as to obtain washed nucleated red blood cells. Preferably, said antibody is an antibody specifically recognizing CD71.

The inventors found that using a method for separating nucleated red blood cells according to embodiments of the present invention, nucleated red blood cells, particularly fetal nucleated red blood cells; can be effectively separated from peripheral blood. Thus, the present invention provides a method for separating fetal nucleated red blood cells from peripheral blood which is simple and easy to operate. It is readily understood by those skilled in the art that in the process of separating nucleated red blood cells, other steps can also be included. For example, according to particular examples of the present invention, the method for separating nucleated red blood cells comprises: taking an appropriate amount of peripheral blood of a pregnant woman, performing anticoagulation with an anticoagulant agent, diluting the blood sample proportionally with 0.1 M PBS free of calcium ions and magnesium ions, placing the diluted sample slowly on a reagent for density gradient centrifugation, and performing density gradient centrifugation at room temperature. After centrifugation, a layer of monocytes can be observed, this layer of cells is pipetted out carefully, transferred into a new centrifuge tube, re-suspended with 3 volumes of PBS buffer containing 1% BSA, and centrifuged again at room temperature, the supernatant is discarded, the obtained cell precipitate is further washed with the same method twice to remove the residual density gradient liquid, and finally, the cell precipitate is re-suspended in PBS containing 0.1% BSA and pipetted uniformly. Then magnetic beads carrying an antibody are added at a proportion of 20 microliters/$10^6$ cells, and centrifuged after standing at 4°C, the supernatant is discarded, and the precipitate is re-suspended in PBS containing 0.1% BSA; and a magnetic bead sorting system is assembled. A sorting column is moistened with 500 microliters of PBS buffer containing 0.1% BSA, and after the liquid is emptied, the cells to be sorted are loaded onto the column, and the effluent liquid is collected and labeled as negative cells. The tube is moistened with PBS containing 0.1% BSA, and after the liquid is emptied, the same is repeated twice. PBS/EDTA/BSA is added into the sorting column, and after the liquid is emptied, same is repeated once again. Finally, PBS containing 0.1% BSA is added into the sorting column, the magnetic field is taken away, the liquid is washed into a new centrifuge tube, and nucleated red blood cells are obtained.

**S20: after fetal nucleated red blood cells are separated from a sample from a pregnant woman, at least a part of the genome of the nucleated red blood cells can be sequenced, and thereby a sequencing result corresponding to sequencing objects can be obtained.**

According to embodiments of the present invention, after the fetal nucleated red blood cells are separated, at least a part of the genome of the nucleated red blood cells can be sequenced. Those skilled in the art can select sequencing objects of the genome of the nucleated red blood cells according to genes of interest, and thereby obtain a sequencing result corresponding to these sequencing objects. According to embodiments of the present invention, those skilled in the art can adopt any known method to select the sequencing objects, e.g., can select only several chromosomes therein. It is readily understood by those skilled in the art that the whole genome of the nucleated red blood cells can also be sequenced directly, and that after a sequencing result is obtained, sequencing data from a specific site are selected from the sequencing result for further analysis (see details hereinafter). For convenience, the following example illustrates the sequencing of the whole genome of nucleated red blood cells.

According to embodiments of the present invention, after the nucleated red blood cells are obtained, the method for sequencing the whole genome of the nucleated red blood cells is not particularly limited. According to an embodiment of the present invention, sequencing the whole genome of the nucleated red blood cells further comprises: firstly, amplifying the whole genome of the nucleated red blood cells to obtain an amplified whole genome; subsequently, constructing a whole-genome sequencing library using the amplified whole genome; and finally, sequencing the whole-genome sequencing library, so as to obtain a sequencing result containing a plurality of sequencing data. Thus, the information from the whole genome of the nucleated red blood cells can be effectively acquired, which thereby further improves the efficiency of determining whether a genomic abnormality exists in the nucleated red blood cells.

Those skilled in the art can select different methods for constructing a whole-genome sequencing library according to the specific protocol for the applied genomic sequencing technique. For details of the construction of the whole-genome sequencing library, see the directive instructions provided by the manufacturer of the sequencer, e.g. the Illumina Corporation. For example, see Multiplexing Sample Preparation Guide (Part#1005361; Feb 2010) or Paired-End SamplePrep Guide (Part#1005063; Feb 2010) by the Illumina Corporation.

Optionally, a method according to an embodiment of the present invention can further comprise a step of lysing said nucleated red blood cells, so as to release the whole genome of said nucleated red blood cells. According to some examples of the present invention, the method that can be used for lysing nucleated red blood cells and releasing the whole genome is not particularly limited, as long as the method can lyse, preferably fully lyse, the nucleated red blood cells. According to the particular examples of the present invention, alkaline lysis buffer can be used for lysing said nucleated red blood cells and releasing the whole genome of said nucleated red blood cells. The inventors found that in this method, the nucleated red blood cells can be effectively lysed and the whole genome be released, and that when the released whole genome is sequenced, the accuracy rate can be improved, which thereby further improves the efficiency of determining chromosomal aneuploidy in the nucleated red blood cells.

According to embodiments of the present invention, the method for amplifying the whole genome of nucleated red blood cells is not particularly limited. A PCR based method, e.g. PEP-PCR, DOP-PCR and OmniPlex WGA can be utilized, and a non-PCR based method, e.g. MDA (multiple displacement amplification), can also be utilized. According to the particular examples of the present invention, preferably, a PCR based method, e.g. the OmniPlex WGA method, is utilized. Selectable commercialized kits include, but are not limited to, GenomePlex from Sigma Aldrich, PicoPlex from Rubicon Genomics, REPLI-g from Qiagen, illustra GenomiPhi from GE Healthcare, etc. Therefore, according to the particular examples of the present invention, before the sequencing library is constructed, OmniPlex WGA can be utilized to amplify the whole genome of the nucleated red blood cells. The whole genome can be effectively amplified, which thereby further improves the efficiency for determining chromosomal aneuploidy in the nucleated red blood cells.

According to embodiments of the present invention, the method of constructing a whole-genome sequencing library (also sometimes referred to as "nucleic acid library" or "sequencing library" herein) using said amplified whole genome further comprises:

Firstly, fragmenting the amplified whole genome, so as to obtain DNA fragments. According to embodiments of the present invention, the method for fragmenting obtained DNA is not particularly limited. According to some particular examples, the fragmentation can be performed through at least one selected from the group consisting of atomization, ultrasonic shearing method, HydroShear and enzyme digestion treatment. Preferably, the amplified whole genome is fragmented using a covaris ultrasonic shearing device. According to embodiments of the present invention, the DNA fragments obtained after the fragmentation treatment are 200-400 bp, preferably 350 bp, in length. The inventors found that the obtained DNA fragments of this length can be effectively used for the construction of the nucleic acid library and subsequent manipulation.

After the DNA fragments are obtained, end repair can be performed on the obtained DNA fragments, so as to obtain end-repaired DNA fragments. According to embodiments of the present invention, the end repair can be performed on the DNA fragments using Klenow fragment, T4 DNA polymerase and T4 polynucleotide kinase, wherein the Klenow fragment has 5'-+3' polymerase activity and 3'→5' exonuclease activity, but lacks the 5'--+3' exonuclease activity, which can thus effectively end repair the DNA fragments.

After the end repair is performed on the DNA fragments, bases A can be added to 3' ends of the end-repaired DNA fragments, so as to obtain DNA fragments with the sticky end A. According to some particular examples of the present invention, bases A can be added to 3' ends of the end-repaired DNA fragments using the Klenow fragment (3'-5' exo-), *i.e.* the Klenow fragment lacking the 3'→5' exonuclease activity, to thus effectively obtain the DNA fragments with the sticky end A.

After bases A are added to the end, the DNA fragments with the sticky end A can be ligated to an adapter, so as to obtain a ligation product. According to embodiments of the present invention, the DNA fragments with the sticky end A can be ligated to the adapter using T4 DNA ligase, to thus effectively obtain the ligation product. According to embodiments of the present invention, a tag can be further included in the adapter, and thus whole-genome sequencing libraries of a plurality of nucleated red blood cell samples can be constructed simultaneously in a convenient manner, and the sequencing libraries of the plurality of samples are combined and sequenced simultaneously. Thus, a high-throughput sequencing platform can be fully utilized to save time and reduce the sequencing cost.

After the ligation product is obtained, PCR amplification is performed on said ligation product, so as to obtain a second amplification product; and said second amplification product is purified and recovered, so as to obtain a recovered product, and said recovered product forms said whole-genome sequencing library.

After the whole-genome sequencing library is constructed, according to embodiments of the present invention, said whole-genome sequencing library can be sequenced. It is readily understood by those skilled in the art that the sequencing step in the present invention can be performed through any sequencing method, which includes, but is not limited to, the dideoxy chain-termination method; preferably high-throughput sequencing methods. Thus, the high throughput and deep sequencing characteristics of these sequence devices can be utilized to further improve the efficiency of determining chromosomal aneuploidy in the nucleated red blood cells. Said high-throughput sequencing methods include, but are not limited to, the second-generation sequencing technique or the single molecule sequencing technique.

Second-generation sequencing platforms (Metzker ML. Sequencing technologies-the next generation. Nat Rev Genet. 2010 Jan; 11(1):31-46) include, but are not limited to, Illumina-Solexa (GA™, HiSeq2000™, etc.), ABI-Solid and Roche-454 (pyrosequencing) sequencing platforms. Platforms (techniques) for the single molecule sequencing include, but are not limited to, the true single molecule sequencing technique (True Single Molecule DNA sequencing) from the Helicos Corporation, the single molecule real-time sequencing technique (SMRT™) from the Pacific Biosciences Corporation, and the nanopore sequencing technique from the Oxford Nanopore Technologies Corporation, etc. (Rusk, Nicole (2009-04-01). Cheap Third-Generation Sequencing. Nature Methods 6 (4): 244-245).

With the continuous development of the sequencing technology, those skilled in the art would understand that the whole-genome sequencing can also be performed using other sequencing methods and devices. According to embodiments of the present invention, the lengths of sequencing data obtained by the whole-genome sequencing are not particularly limited. According to a particular example of the present invention, the average length of said plurality of sequencing data is about 50 bp. The inventors discovered that when the average length of the sequencing data is about 50 bp, the analysis of the sequencing data can be greatly facilitated, the analysis efficiency is improved, and at the same time, the cost of analysis can be significantly reduced. Thus, the efficiency of determining chromosomal aneuploidy in the nucleated red blood cells is further improved, and the cost of determining chromosomal aneuploidy in the nucleated red blood cells is reduced. The term "average length" as used herein refers to the average value of the numerical values of the lengths of all the sequencing data.

S30: after the sequencing result is obtained, whether a genomic abnormality exists in the nucleated red blood cells is determined based on the obtained sequencing result.

The term "genomic abnormality" as used herein should be understood in a broad sense, which can refer to any change in the genomic sequence, e.g., chromosomal aneuploidy, structural variation, single nucleotide mutation and other genetic variations (www.en.wikipedia.org/wiki/Genetic_variation), which can also be a change in a genomic modification site, *e.g.* the methylation level, etc. According to embodiments of the present invention, the studied genomic abnormality is at least one selected from the group consisting of chromosomal aneuploidy and a mutation in a predetermined region. In the embodiments of the present invention, the mutation in a predetermined region refers to structural variation (www. en.wikipedia.org/wiki/Structural_variation) or single nucleotide mutation (SNP, www.en.wikipedia.org/wiki/Single-nucleotide_polymorphism).

According to embodiments of the present invention, determining a mutation in a predetermined region in the genome can further comprise:

Firstly, determining the nucleic acid sequence of the predetermined region in said nucleated red blood cells based on the sequencing result. Those skilled in the art can utilize any known method to determine the nucleic acid sequence of the predetermined region. For example, a known method can be utilized to assemble sequencing data from a specific region in the sequencing result, thereby obtaining the nucleic acid sequence from a predetermined sequence.

After the nucleic acid sequence from the predetermined sequence is obtained, the nucleic acid sequence of the predetermined region in said nucleated red blood cells is aligned to a control nucleic acid sequence; preferably, said control nucleic acid sequence is a normal human genomic sequence. Subsequently, on the basis of a result of the alignment, it can be determined whether an abnormality exists in the predetermined region in the nucleated red blood cells. According to embodiments of the present invention, the mutation in a predetermined region that can be detected by the method includes at least one selected from the group consisting of insertion mutation, deletion mutation, substitution mutation, inversion mutation, copy number variation, translocation mutation and single nucleotide polymorphism.

Referring to Figure 2, according to an embodiment of the present invention, the method for determining chromosomal aneuploidy comprises the steps of:

S100: firstly, sequencing the whole genome of the nucleated red blood cells to obtain a first sequencing result. Description on sequencing the whole genome has been detailed above, thus is not repeated.

S200: dividing the known sequence of a first chromosome into windows

In order to analyze the sequencing data of the nucleated red blood cells, the known sequence of the first chromosome is first divided into windows, and each of these windows independently has a predetermined length of the sequences, respectively. According to an embodiment of the present invention, the lengths of sequences within these windows can be the same, can also be different, and are not particularly limited. According to an embodiment of the present invention, the predetermined lengths of sequences within said plurality of windows are the same. Preferably, the predetermined lengths of sequences within said plurality of windows are all 60 kB. Thus, the efficiency of determining chromosomal aneuploidy in the nucleated red blood cells can be improved. Those skilled in the art can select the sequence range of the first chromosome covered by the windows as required.

S300: determining the number of sequencing data falling in each window

After the sequencing data are obtained, the obtained sequencing data are aligned to the known sequence of the first chromosome to thereby divide the obtained sequencing data into the windows with predetermined lengths, respectively. Those skilled in the art would understand that any known method and means can be used to perform the sequence alignment and to calculate the total number of these sequencing data. For example, software provided by the manufacturer of the sequencer can be adopted to perform analysis, *e.g.* SOAP v2.20. According to an embodiment of the present invention, said sequencing data falling in each window are uniquely aligned sequencing data. Thus, through screening of the sequencing data, the efficiency of determining chromosomal aneuploidy in the nucleated red blood cells can be further improved. The term "uniquely aligned sequencing data," also referred to as "unique read," as used herein refers to sequencing data that can be matched perfectly and aligned successfully only once with a reference genome when the sequencing data are aligned to a known chromosomal sequence, *e.g.* the human genome Hg19.

The term "first chromosome" as used herein should be understood in a broad sense, which can refer to any target chromosome that is expected to be studied. The number thereof is not limited to only one chromosome, more and even all of the chromosomes can be analyzed simultaneously. According to embodiments of the present invention, the first chromosome can be any chromosome selected from human chromosomes 1-23. According to embodiments of the present invention, preferably, the "first chromosome" is at least one selected from the group consisting of human chromosome 21, chromosome 18, chromosome 13, X chromosome and Y chromosome. Thus, common human chromosomal diseases can be effectively determined, for example, for prognosis of fetal genetic diseases. Therefore, the method for determining chromosomal aneuploidy in nucleated red blood cells according to embodiments of the present invention can be very effectively applied in pre-implantation screening (PGS) and pre-implantation diagnosis (PGD) in the field of *in vitro* reproduction, prenatal testing on fetal nucleated cells, etc. Thus, it can be rapidly predicted whether a chromosomal abnormality exists in a fetus through simple isolation of nucleated red blood cells, which can avoid the case that the fetus suffers from a serious genetic disease. The term "can be aligned with the first chromosome" as used herein refers to that through the alignment of sequencing data to the known sequence of the first chromosome of the reference genome, the sequence data can be aligned with the known sequence of the first chromosome, thereby it is determined that these sequencing data are derived from the first chromosome.

S400: determining a first parameter based on the number of sequencing data falling in each window

In the sequencing result of the whole-genome sequencing of the nucleated red blood cells 14, the number of sequencing data for a specific chromosome has a positive correlation with the content of this chromosome in the whole genome. Therefore, through analysis of the number of sequencing data derived from a specific chromosome in the sequencing result and the total number derived from the whole-genome sequencing, the specific chromosome can be effectively analyzed. To this end, a first parameter can be determined through analysis of the number of sequencing data falling in each window of the first chromosome. In order to make the first parameter capable of truly reflecting whether aneuploidy exists in the first chromosome, according to an embodiment of the present invention, the first parameter based on the number of sequencing data falling in each window is determined by a method further comprises : setting a predetermined weighting coefficient (also referred to as "weighted coefficient" herein sometimes) for the number of sequencing data falling in each window, respectively; and according to the weighting coefficient, performing weighted averaging on the number of sequencing data falling in each window to obtain the median of said first chromosome, the obtained median forming the first parameter of the first chromosome. The inventors found that the first parameter obtained in this way can effectively embody the number of sequencing data from the first chromosome in the sequencing data, and thereby the efficiency of determining chromosomal aneuploidy can be further improved.

According to embodiments of the present invention, the weighting coefficient is set for being capable of eliminating data distortion caused by some errors that may exist in the sequencing process. According to an example of the present invention, the predetermined weighting coefficient is obtained by associating the number of sequencing data falling in each window with the GC content of the respective window. Thus, resulting errors due to bias of the sequencing technique towards a region with a high GC content can be effectively eliminated. For example, according to an embodiment of the present invention, the weighting coefficient can be obtained through the following method:

Firstly, each chromosome of the whole human genome is divided into windows with a fixed length of 60 kB respectively, and the starting position and ending position of each window are recorded and the GC average value (recorded as $GC_{ref}$) thereof is obtained by statistics. After sequencing data of a sample to be tested are obtained, the sequences in the sequencing result are aligned to the genome. Sequencing data that are matched perfectly and aligned successfully only once, *i.e.* uniquely aligned sequencing data, are taken out, and the information about the sites of all the uniquely aligned sequencing data is obtained. The number of uniquely aligned sequencing data (recorded as $UR_{sample}$) in each window for each chromosome in the genome corresponding to the alignment result of the sample to be tested is counted. The resulting $UR_{sample}$ and $GC_{ref}$ are plotted to obtain Figure 8A. As shown by Figure 8A, the GC bias introduced by the sequencer caused more distribution of sequencing data with GC content roughly in the [0.35, 0.55] region. The discrete points in Figure 8A are fitted with the smooth spline method into a smooth curve to obtain Figure 8B, which is a diagram reflecting the relationship between the GC content and the number of sequencing data. Figure 8B shows all the windows divided by a step length of 1% GC average value. The number of uniquely aligned sequencing data, *i.e.* $M_{fit}$, in a window corresponding to each GC average value can be obtained from fitted data. According to the formula $W_{GC} = M/M_{fit}$, the predetermined weighting coefficient $W_{GC}$ is obtained, and see Figure 8C for the distribution of the weighting coefficient, wherein M is the number of uniquely aligned sequencing data from the sample to be tested the fall in windows of an equal GC average value.

Subsequently, after the first parameter is obtained, it can be determined whether the nucleated red blood cells have aneuploidy for said first chromosome by comparing the first parameter with a predetermined control parameter. The term "predetermined" as used herein should be understood in a broad sense, which can be determined through an experiment in advance, and can also be obtained by conducting a parallel experiment when a biological sample is analyzed. The term "parallel experiment" as used herein should be understood in a broad sense, which not only can refer to simultaneous sequencing and analysis of an unknown sample and a known sample, but also can refer to sequencing and analysis performed successively under the same condition. For example, a first parameter for the sample obtained by testing a nucleated red blood cell sample known to have aneuploidy or a nucleated red blood cell sample known not to have aneuploidy can be used as the control parameter.

In addition, the inventors found that whether the first chromosome has aneuploidy can be determined by comparing and statistically analyzing the numbers of sequencing data of different chromosomes in the same run of sequencing. Therefore, according to an embodiment of the present invention, whether the nucleated red blood cells have aneuploidy for the first chromosome based on the first parameter can be determined by a method further comprises: performing the same treatment on a second chromosome as on the first chromosome, so as to obtain the median of said second chromosome; performing the t value test on the median of the first chromosome and the median of the second chromosome, so as to obtain the difference between said first chromosome and said second chromosome; and comparing the obtained difference with a predetermined first threshold and second threshold, and if the obtained difference is lower than the predetermined thresholds, then determining that the nucleated red blood cells have aneuploidy for the first chromosome, and if the obtained difference is higher than said predetermined thresholds, then determining that the nucleated cells do not have aneuploidy for the first chromosome. The difference between the first chromosome and other chromosomes can also be determined to thereby improve the efficiency of determining chromosomal aneuploidy.

The term "second chromosome" as used herein should be understood in a broad sense, which can refer to any target chromosome that is expected to be studied, the number thereof is not limited to only one chromosome, more and even all the chromosomes other than the first chromosome can be analyzed simultaneously. According to embodiments of the present invention, the second chromosome can be any chromosome among the human chromosomes, and for fetal nucleated red blood cells, the second chromosome is preferably any chromosome selected from human chromosomes 1-23. According to embodiments of the present invention, preferably, the second chromosome is any one selected from the group consisting of chromosomes 1-12 in the human genome. Because chromosomal aneuploidy does not exist in these chromosomes ordinarily, these chromosomes can be effectively used as a reference to test the first chromosome to improve the testing efficiency.

According to an embodiment of the present invention, the following formula is used to perform the t value test on the median of a first chromosome and the median of a second chromosome,

$$T_{i,j} = \frac{\mu_i - \mu_j}{\sqrt{\dfrac{\sigma_i^2}{n_i} + \dfrac{\sigma_j^2}{n_j}}} \quad \text{(formular I)}$$

where $T_{i,j}$ represents the difference between the first chromosome and the second chromosome, $\mu_i$ represents the median of the first chromosome, $\mu_j$ represents the median of the second chromosome, $\sigma_i$ represents the standard deviation of the distribution of the number of sequencing data in each window in the first chromosome, $\sigma_j$ represents the standard deviation of the distribution of the number of sequencing data in each window in the second chromosome, $n_i$ represents the number of the windows in the first chromosome, and $n_j$ represents the number of the windows in the second chromosome.

According to embodiments of the present invention, the value of the predetermined thresholds can be obtained by experience, or a corresponding t test value obtained by testing in advance a nucleated red blood cell sample known to have aneuploidy or a nucleated red blood cell sample known not to have aneuploidy is used as a threshold. Preferably, the predetermined first threshold is -4 or less, and the second threshold is -3.5 or greater.

In contrast to chromosomal euploidy, the term "aneuploidy" as used herein refers to one or several chromosomes being missing or added to the genome thereof. Generally, there are two chromatins for each chromosome in a normal cell. However, gametes with abnormal numbers of chromosomes are formed due to nondisjunction or too early disjunction of a pair of homologous chromosomes during meiosis, and the union of such gametes with each other or with normal gametes will generate various aneuploid cells. In addition, aneuploid cells, such as tumor cells with a very high mutation rate, etc., can also be generated during somatic cell division.

## 2. System for determining whether a genomic abnormality exists in nucleated red blood cells

According to yet another aspect of the present invention, the present invention provides a system 1000 for determining whether a genomic abnormality exists. Referring to Figure 3, according to an embodiment of the present invention, the system 1000 comprises: a nucleated red blood cell separation device 100, a sequencing device 200 and a sequencing result analysis device 300. According to an embodiment of the present invention, the nucleated red blood cell separation device 100 is used for separating fetal nucleated red blood cells from a sample from a pregnant woman. The sequencing device 200 is used for sequencing at least a part of the genome of the nucleated red blood cells, so as to obtain a sequencing result. The sequencing result analysis device 300 is connected to the sequencing device 200, so as to receive the sequencing result from the sequencing device 200, and determine whether a genomic abnormality exists in the separated nucleated red blood cells based on the obtained sequencing result.

According to an embodiment of the present invention, the nucleated red blood cell separation device 100 can further comprise a monocyte separation unit 101 and a magnetic enrichment unit 102. According to an embodiment of the present invention, the monocyte separation unit 101 is suitable for performing gradient centrifugation on the sample from a pregnant woman using a density gradient reagent, so as to obtain monocytes, wherein the sample from a pregnant woman is peripheral blood of the pregnant woman. The magnetic enrichment unit 102 is connected to the monocyte separation unit 101, and is suitable for separating nucleated red blood cells from the monocytes using magnetic beads carrying an antibody, wherein said antibody specifically recognizes an antigen on the surface of the nucleated red blood cells. Thus, assisted by the nucleated red blood cell separation device 100, the nucleated red blood cells can be effectively separated through the following method:

Firstly, gradient centrifugation is performed on the maternal peripheral blood using a density gradient reagent, so as to obtain monocytes. After the monocytes are obtained, nucleated red blood cells are enriched from the obtained monocytes using magnetic beads carrying an antibody, wherein the antibody carried on the magnetic beads specifically recognizes an antigen on the surface of the nucleated red blood cells to thereby bind the nucleated red blood cells to the magnetic beads through the antibody. Subsequently, the nucleated red blood cells can be obtained through magnetic screening.

According to the particular examples of the present invention, the above-mentioned nucleated red blood cell separating device 100 is suitable for performing the following operations: taking an appropriate amount of peripheral blood of a pregnant woman; performing anticoagulation with an anticoagulant agent; diluting the blood sample proportionally with 0.1 M PBS free of calcium ions and magnesium ions; placing the diluted sample slowly on a reagent for density gradient centrifugation; and performing density gradient centrifugation at room temperature. After the centrifugation, a layer of monocytes is observed; this layer of cells is pipetted out carefully, transferred into a new centrifuge tube, re-suspended with 3 volumes of PBS buffer containing 1% BSA, and centrifuged again at room temperature; the supernatant is discarded; the obtained cell precipitate is further washed with the same method twice to remove the residual density gradient liquid; and finally, the cell precipitate is re-suspended in PBS containing 0.1% BSA and pipetted uniformly. Then magnetic beads carrying an antibody are added to the suspended cells at a proportion of 20 microliters/$10^6$ cells; the mixture of the cells and beads are centrifuged after standing at 4°C; the supernatant is discarded; and the precipitate is re-suspended in PBS containing 0.1% BSA. A magnetic bead sorting system is assembled: a sorting column is moistened with 500 microliters of PBS buffer containing 0.1% BSA; after the liquid is emptied, the cells to be sorted are loaded onto the column; and the effluent liquid is collected and labeled as negative cells. The tube is moistened with PBS containing 0.1% BSA, and after the liquid is emptied, same is repeated twice; PBS/EDTA/BSA is added into the sorting column, and after the liquid is emptied, same is repeated once. Finally, PBS containing 0.1% BSA is added into the sorting column; the magnetic field is taken away; the liquid is washed into a new centrifuge tube; and nucleated red blood cells are obtained. The method for separating nucleated red blood cells has been detailed above, and is not repeated.

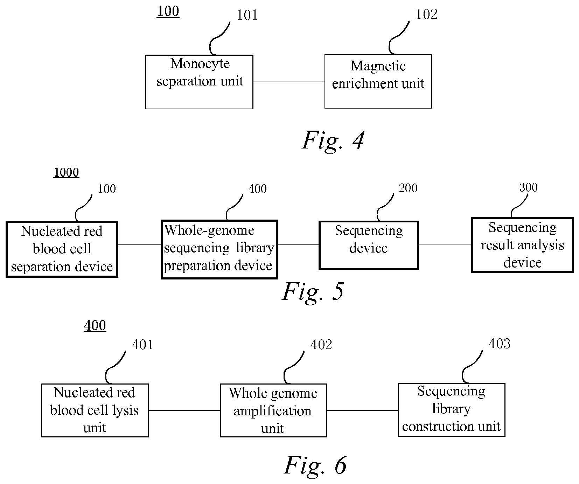

In addition, according to embodiments of the present invention, referring to Figure 5, the system can further comprise a whole-genome sequencing library preparation device 400. According to embodiments of the present invention, the whole-genome sequencing library preparation device 400 is connected to the sequencing device 200, and provides a whole-genome sequencing library for sequencing by the sequencing device 200. Referring to Figure 6, according to an embodiment of the present invention, the whole-genome sequencing library preparation device 400 can further comprise a nucleated red blood cell lysis unit 401, a whole genome amplification unit 402 and a sequencing library construction unit 403. According to embodiments of the present invention, the nucleated red blood cell lysis unit 401 is connected to the nucleated red blood cell separation device 100, and receives and lyses the separated nucleated red blood cells, so as to release the whole genome of the nucleated red blood cells. The whole genome amplification unit 402 is connected to the nucleated red blood cell lysis unit 401, and is used for amplifying the whole genome of the nucleated red blood cells, so as to obtain an amplified whole genome. The sequencing library construction unit 403 is used for receiving the amplified whole genome, and constructing the whole-genome sequencing library using the amplified whole genome. The whole-genome sequencing library preparation device can effectively construct a sequencing library of nucleated red blood cells.

The term "connect" as used herein should be understood in a broad sense, which not only can refer to direct connection, but also can refer to indirect connection, and even the same container or equipment can be used, as long as a functional engagement can be realized. For example, the nucleated red blood cell lysis unit 302 and the whole genome amplification unit 303 can be in the same equipment, *i.e.*, after the lysis of the nucleated red blood cells are realized, the whole genome amplification treatment can be performed in the same equipment or container, and the released whole genome needs not to be delivered to other equipment or containers, as long as the condition (including the reaction condition and the composition of the reaction system) in the equipment is converted into that suitable for performing the whole genome amplification reaction. Accordingly, a functional engagement of the nucleated red blood cell lysis unit 302 and the whole genome amplification unit 303 is realized, which is considered to be encompassed by the term "connect".

Those skilled in the art can select different methods and equipment for constructing a whole-genome sequencing library according to a specific protocol for the applied genomic sequencing technique, and for details of the construction of the whole-genome sequencing library, the reference can be made to the directive rules provided by the manufacturer of the sequencer, e.g., the Illumina Corporation. According to an embodiment of the present invention, the whole genome amplification unit 303 comprises a device suitable for amplifying said whole genome using the OmniPlex WGA method. Thus, the whole genome can be effectively amplified, and thereby the efficiency of determining a genomic abnormality in the nucleated red blood cells is further improved.

According to an embodiment of the present invention, the whole-genome sequencing device 100 includes at least one selected from the group consisting of illumina-Solexa, ABI-Solid, Roche-454 and a single molecule sequencing device. Thus, these sequencing devices' characteristics of high throughput and deep sequencing can be used, and thereby the efficiency of determining chromosomal aneuploidy in the nucleated red blood cells is further improved. It is readily understood by those skilled in the art that the whole-genome sequencing can also be performed using other sequencing methods and devices, *e.g.*, the third-generation sequencing technique, and more advanced sequencing techniques that may be developed afterwards. According to embodiments of the present invention, the lengths of sequencing data obtained by the whole-genome sequencing are not particularly limited.

As previously mentioned, according to embodiments of the present invention, after the genomic sequencing result of the nucleated red blood cells is obtained, chromosomal aneuploidy can be analyzed. Therefore, according to embodiments of the present invention, the sequencing result analysis device 300 can be suitable for executing the following operations: firstly, dividing the known sequence of a first chromosome into a plurality of windows, the plurality of windows independently having a predetermined length, respectively; subsequently, aligning the sequencing data in said sequencing result to the known sequence of the first chromosome, so as to obtain the number of sequencing data falling in each window; finally, on the basis of obtaining the number of sequencing data falling in each window, determining a first parameter; and on the basis of said first parameter, determining whether said nucleated red blood cells have aneuploidy for said first chromosome. Whether the nucleated red blood cells have chromosomal aneuploidy can be effectively determined using the system 1000.

According to an embodiment of the present invention, the sequencing result analysis device 300 further comprises a sequence alignment unit (not shown in the figures). The sequence alignment unit is used for aligning the sequencing result to the information about the known genomic sequence, so as to obtain all sequencing data that can be aligned with the reference genome and to obtain sequencing data from the first chromosome. Thus, sequencing data from a specific chromosome can be effectively determined, and thereby the efficiency of determining chromosomal aneuploidy in the nucleated red blood cells is further improved. The term "first chromosome" used herein should be understood in a broad sense, same can refer to any target chromosome that is expected to be studied, the number thereof is not limited to only one chromosome, more and even all the chromosomes can be analyzed simultaneously. According to embodiments of the present invention, the first chromosome can be any chromosome among the human chromosomes, *e.g.,* can be at least one selected from the group consisting of human chromosome 21, chromosome 18, chromosome 13, X chromosome and Y chromosome. Thus, common human chromosomal diseases can be effectively determined, for example, fetal genetic diseases can be predicted.

Therefore, the method for determining chromosomal aneuploidy in nucleated red blood cells according to embodiments of the present invention can be very effectively applied in pre-implantation screening (PGS) and pre-implantation diagnosis (PGD) in the field of in vitro reproduction, and prenatal testing on fetal nucleated cells, etc. It can be rapidly predicted whether a chromosomal abnormality exists in a fetus through the simple extraction of nucleated red blood cells, which avoids the case that the fetus suffers from a serious genetic disease.

The first parameter and other characteristics have been detailed above, and are not repeated here. The following should be noted:

According to an embodiment of the present invention, the sequencing result analysis device 300 can further comprise a unit which is suitable for determining a first parameter based on the number of sequencing data falling in each window, via the following steps: setting a predetermined weighting coefficient for the number of sequencing data falling in each window, respectively; and according to said weighting coefficient, and performing weighted averaging on said number of sequencing data falling in each window to obtain the median of said first chromosome, which forms the first parameter of said first chromosome.

According to an embodiment of the present invention, the sequencing result analysis device 300 can further comprise a unit which is suitable for determining whether said nucleated red blood cells have aneuploidy for said first chromosome based on the first parameter, through the following steps: performing the same treatment on a second chromosome as on said first chromosome, so as to obtain the median of said second chromosome; performing the t value test on the median of said first chromosome and the median of said second chromosome, so as to obtain the difference between said first chromosome and said second chromosome; and comparing said difference with a predetermined threshold, and if said difference is lower than said predetermined threshold, then determining that said nucleated red blood cells have aneuploidy for the first chromosome.

According to an embodiment of the present invention, the sequencing result analysis device 300 can further comprise a unit which is suitable for using the following formula to perform the t value test on the median of said first chromosome and the median of said second chromosome,

$$T_{i,j} = \frac{\mu_i - \mu_j}{\sqrt{\dfrac{\sigma_i^2}{n_i} + \dfrac{\sigma_j^2}{n_j}}}$$

where $T_{i,j}$ represents the difference between said first chromosome and said second chromosome; $\mu_i$ represents the median of the first chromosome, $\mu_j$ represents the median of the second chromosome; $\sigma_i$ represents the standard deviation of the distribution of the number of sequencing data in each window in the first chromosome; $\sigma_j$ represents the standard deviation of the distribution of the number of sequencing data in each window in the second chromosome; $n_i$ represents the number of the windows in the first chromosome; and $n_j$ represents the number of the windows in the second chromosome.

In addition, according to embodiments of the present invention, the sequencing result analysis device 300 is suitable for determining whether a mutation exists in a predetermined region in the genome. Therefore, according to an embodiment of the present invention, the sequencing result analysis device 300 can further comprise: a determination unit for nucleic acid sequence, an alignment unit and an abnormality determination unit. The determination unit for a predetermined nucleic acid region is suitable for determining the nucleic acid sequence of the predetermined region in said nucleated red blood cells based on the sequencing result. The alignment unit is connected to said nucleic acid sequence determination unit, and is suitable for aligning the nucleic acid sequence of the predetermined region in said nucleated red blood cells to a control nucleic acid sequence. Preferably, the control nucleic acid sequence is stored in the alignment unit. More preferably, the control nucleic acid sequence is the normal human genome sequence. The abnormality determination unit is connected to said alignment unit, and is suitable for determining whether an abnormality exists in the predetermined region in said nucleated red blood cells based on a result of said alignment. The method and detail for determining a mutation in a predetermined region have been detailed above, and are not repeated here.

Other characteristics and advantages described in relevant methods above are also suitable for the system for determining whether an abnormality exists in nucleated red blood cells, and are not repeated here.

**3. Method for determining the genomic sequence of fetal nucleated red blood cells**

Yet another aspect of the present invention relates to a method for determining the genomic sequence of fetal nucleated red blood cells, which comprises the following steps:

Firstly, fetal nucleated red blood cells are separated from a sample from a pregnant woman. After the fetal nucleated red blood cells are obtained, at least a part of the genome of the separated nucleated red blood cells is sequenced, so as to obtain a sequencing result. Finally, on the basis of the obtained sequencing result, the genomic sequence of the fetal nucleated red blood cells is determined.

The expression "genomic sequence" as used herein should be understood broadly, *i.e.* it can be the sequence of the whole genome, and can also be the sequence of a part of the genome. Separating fetal nucleated red blood cells from a sample from a pregnant woman and sequencing at least a part of the genome of the nucleated red blood cells have been detailed above, and are not repeated here. What needs to be noted is:

According to an embodiment of the present invention, the sample from a pregnant woman is peripheral blood of the pregnant woman. According to another embodiment of the present invention, the gestational age of said pregnant woman is 12-20 weeks. According to yet another embodiment of the present invention, separating fetal nucleated red blood cells from peripheral blood of said pregnant woman further comprises: performing gradient centrifugation on said peripheral blood using a density gradient reagent, so as to obtain monocytes; and separating nucleated red blood cells from said monocytes using magnetic beads carrying an antibody, wherein said antibody specifically recognizes an antigen on the surface of the nucleated red blood cells. According to an embodiment of the present invention, said density gradient reagent is polysucrose, optionally, said gradient centrifugation is performed at $800 \times$ g for 30 minutes. According to an embodiment of the present invention, after said monocytes are obtained and before said nucleated red blood cells are separated using the magnetic beads carrying anti-CD71 antibody, the process further comprises washing said monocytes using PBS buffer containing 1% BSA, so as to remove the residual density gradient reagent, preferably, said PBS buffer being free of calcium ions and magnesium ions. According to an embodiment of the present invention, washing said monocytes using PBS buffer containing 1% BSA further comprises: mixing said monocytes with said PBS buffer containing 1% BSA, so as to obtain a suspension containing monocytes; and centrifuging said suspension containing monocytes, preferably, at $200 \times$ g for 5 minutes, and discarding the supernatant, so as to obtain washed nucleated red blood cells. According to an embodiment of the present invention, said antibody is an antibody specifically recognizing CD71. According to an embodiment of the present invention, a single nucleated red blood cell is sequenced. According to an embodiment of the present invention, the whole genome of said nucleated red blood cells is sequenced. According to an embodiment of the present invention, sequencing the whole genome of said nucleated red blood cells further comprises: amplifying the whole genome of said nucleated red blood cells to obtain an amplified whole genome; constructing a whole-genome sequencing library using said amplified whole genome; and sequencing said whole-genome sequencing library, so as to obtain a sequencing result consisting of a plurality of sequencing data. According to an embodiment of the present invention, the whole genome of said nucleated red blood cells is amplified through the OmniPlex WGA method. According to an embodiment of the present invention, constructing a whole-genome sequencing library using said amplified whole genome further comprises: fragmenting said amplified whole genome, so as to obtain DNA fragments; performing end repair on said DNA fragments, so as to obtain end-repaired DNA fragments; adding bases A to 3' ends of said end-repaired DNA fragments, so as to obtain DNA fragments with the sticky end A; ligating said DNA fragments with the sticky end A to an adapter, so as to obtain a ligation product; performing PCR amplification on said ligation product, so as to obtain a second amplification product; and purifying and recovering said second amplification product, so as to obtain a recovered product, and said recovered product forming said whole-genome sequencing library. According to an embodiment of the present invention, fragmenting said amplified whole genome is performed through a Covaris shearing device. According to an embodiment of the present invention, the lengths of said DNA fragments are about 350 bp. According to an embodiment of the present invention, performing end repair on said DNA fragments is performed using Klenow fragment, T4 DNA polymerase and T4 polynucleotide kinase, and said Klenow fragment has 5'→3' polymerase activity and 3'→5' exonuclease activity, but lacks 5'→3' exonuclease activity. According to an embodiment of the present invention, adding bases A to 3' ends of said end-repaired DNA fragments is performed using Klenow fragment (3'-5' exo-). According to an embodiment of the present invention, ligating said DNA fragments with the sticky end A to an adapter is performed using T4 DNA ligase. According to an embodiment of the present invention, said sequencing is performed using at least one selected from the group consisting of Hiseq2000, SOLiD, 454 and a single molecule sequencing device. The advantages of these characteristics have been detailed above, and are not repeated.

Other characteristics and advantages described in the method for determining whether a genomic abnormality exists in the chromosomes of nucleated red blood cells above are also suitable for the method for determining the genomic sequence of fetal nucleated red blood cells, and are not repeated here.

The present invention is described below through particular embodiments It needs to be noted that these embodiments are only for the purpose of illustration, thus should not be construed to limit the present invention in any way.

**Embodiment 1:**

Experimental materials

Peripheral blood samples were obtained from pregnant women with high risk of Downs Syndrome infants, all of whom already had a clinical outcome. If not specially indicated, all the other test materials were reagents prepared by conventional methods in the art or commercially available reagents.

Experimental procedure

1. Separating nucleated red blood cells

Peripheral blood of pregnant women (3 mL) was taken. EDTA was selected as an anticoagulant agent. The blood sample was diluted with 0.1 M phosphate buffer solution (PBS) free of $Ca^{2+}$ and $Mg^{2+}$ at a proportion of 1:1. The diluted sample was placed slowly on 3 mL of the Ficoll reagent (a product of the Sigma Corporation, US) with a density of 1.077, and density gradient centrifugation was performed at room temperature, 800 g×30 min. After centrifugation, a layer of monocytes was observed. The layer of cells was pipetted out carefully, and transferred into a new 1.5 mL centrifuge tube. The obtained monocytes were re-suspended with 3 volumes of PBS containing 1% BSA, and centrifuged at room temperature 200 g×5 min. The supernatant was discarded, and the cell precipitate was further washed with the same method twice to remove the residual density gradient liquid. Finally, the cell precipitate was re-suspended in 300 microliters of PBS containing 0.1% BSA and pipetted uniformly. The cells were counted, and then were added magnetic beads carrying anti-CD71 antibody (the Miltenyi Biotec Corporation, Germany) at a proportion of 20 microliters/$10^6$ cells, and stood at 4°C for 15 min. Centrifugation was performed, 300 g×10 min. The supernatant was discarded, and the precipitate was re-suspended in 500 microliters of PBS containing 0.1% BSA. A magnetic bead sorting system (the Miltenyi Biotec Corporation, Germany) was assembled. A sorting column was moistened with 500 microliters of PBS containing 0.1% BSA. After the PBS was emptied, the cells to be sorted were loaded onto the column, and the effluent liquid was collected and labeled as negative cells. The tube was moistened with 500 microliters of PBS containing 0.1% BSA, and after the liquid was emptied, the same procedure was repeated twice. PBS/EDTA/BSA (500 microliters) was added into the sorting column, and after the liquid was emptied, same procedure was repeated once. Finally, 1 mL of PBS containing 0.1% BSA was added into the sorting column, the sorting column was taken away from the magnetic field, and the cells were washed into a 15 mL tube and labeled as positive cells. The positive cells were centrifuged and concentrated, only about 100 microliters of the lowest layer was retained and ready for use, and the obtained nucleated red blood cells were labeled as GP9.

2. Constructing whole-genome sequencing libraries

Whole genome amplification was performed on all of the separated nucleated red blood cells. The GenomePlex Single Cell Whole Genome Amplification Kit was selected to perform the whole genome amplification in this experiment. The operational procedure was performed according to the instructions provided by the manufacturer, the Sigma Corporation. The amplification product was sheared by a Covaris shearing device in strict accordance with the instructions accompanying the shearing device to obtain a sheared product, *i.e.,* DNA fragments, with the sheared main band concentrated at around 350 bp.

End repair was performed on the sheared product and bases A were added to an end using the specific process as follows:

The reaction for end repair was performed with the following system:

| | |
|---|---|
| 10×T4 polynucleotide kinase buffer | 10 μL |
| dNTPs (10 mM) | 4 μL |
| T4 DNA polymerase | 5 μL |
| Klenow fragment | 1 μL |
| T4 polynucleotide kinase | 5 μL |
| Sheared product DNA fragments | 30 μL |
| ddH$_2$O added up to 100 μL | |

After reaction at 20°C for 30 minutes, the end-repaired product was recovered using the PCR purification kit (QIAGEN). The obtained product was finally dissolved in 34 μL of EB buffer.

The reaction for adding bases A to an end was accomplished with the following system:

| | |
|---|---|
| 10× Klenow buffer | 5 μL |
| dATP (1 mM) | 10 μL |
| Klenow (3'-5'exo⁻) | 3 μL |
| DNA | 32 μL |

After incubation at 37°C for 30 minutes, DNA with base A added to an end was purified by the MinElute® PCR purification kit (QIAGEN), and the product was dissolved in 12 μL of EB.

The ligation reaction for the adapter was as follows:

| | |
|---|---|
| 2x Rapid DNA ligation buffer | 25 μL |
| PEI A adapter oligomix (20 μM) | 10 μL |
| T4 DNA ligase | 5 μL |
| DNA with bases A added to an end | 10 μL |

After reaction at 20°C for 15 minutes, the ligation product was recovered using the PCR purification kit (QIAGEN). The product was finally dissolved in 32 μL of EB buffer.

The PCR reaction system was prepared in a 0.2 mL PCR tube:

| | |
|---|---|
| Sample | 10 μL |
| Phusion DNA polymerase Mix | 25 μL |
| PCR primer* (10 pmol/|iL) | 1 μL |
| Index N primer** (10 pmol/μL) | 1 μL |
| UltraPureTM water | 13 μL |

\* The sequence of the PCR primer was

AATGATACGGCGACCACCGAGATCTACACTCTTTCCCTACACGACGCTCTTCCGATCT

\*\*The sequence of the index N primer was

CAAGCAGAAGACGGCATACGAGATCGTGATGTGACTGGAGTTCAGACGTGTGCTCTTCCGATCT

The reaction program was as follows:

$$
\begin{array}{ll}
98°C & 30\ s \\
98°C & 10\ s \\
65°C & 30\ s \\
72°C & 30\ s
\end{array}
\left.\vphantom{\begin{array}{l} 1\\2\\3\\4 \end{array}}\right\}\ 10\ cycles
$$

$$
\begin{array}{ll}
72°C & 5\ min \\
4°C & maintained
\end{array}
$$

The PCR product was recovered using the PCR purification kit (QIAGEN). The sample was finally dissolved in 22 μL of EB buffer.

Among them, index N was a unique tag sequence of 8 bp in each library respectively. The constructed libraries were tested by Agilent®Bioanalyzer 2100, and the results were shown in Figure 8. As shown in Figure 8, the range of distribution of fragments of the constructed libraries met the requirements. The libraries were further quantified by the Q-PCR method respectively. After being qualified, the libraries were mixed in the same lane in a flow cell to perform in silico sequencing, and in order to save the cost, single-end sequencing was selected. In this example, illumina® HiSeq2000™ sequencing instrument and a method therefor were selected to perform sequencing, where setting of parameters and the operational method for the instrument were performed in strict accordance with the operational manual provided by the illumina® manufacturer (available from www.illumina.com/support/documentation.ilmn). In this example, the HiSeq2000TM sequencer was used, and the number of sequencing cycles was PE91index (*i.e.* pair-end 91bp index sequencing).

3. Data analysis

The sequencing results obtained by sequencing are shown in Table 1, where the total number of sequencing data (reads) obtained by sequencing of the sample GP9 was 13,407,381, the number of those that could be aligned with the reference genome (HG19) was 9,217,701, the alignment rate was 68.70%, the number of sequencing data that could be uniquely aligned with the reference sequence was 7,341,230, and the unique alignment rate was 80%.

Table 1 Sequencing data

| Sample name | Total number of sequencing data | Number of sequencing data that could be aligned | Alignment rate | Number of sequencing data that could be uniquely aligned | Unique alignment rate |
|---|---|---|---|---|---|
| GP9 | 13407381 | 9217701 | 68.70% | 7341230 | 80% |

After preliminary analysis of the data, the sequencing read length was aligned to the reference sequence hg19 using SOAP v2.20, and the alignment method was as follows:
A weighting coefficient was obtained through the following method:
Firstly, each chromosome of the whole human genome was divided into windows with a fixed length of 60 kB respectively, and the starting position and ending position of each window were recorded and the GC average value (recorded as $GC_{ref}$) thereof was obtained by statistics. After sequencing data of a sample to be tested were obtained, the sequences in the sequencing result were aligned to the genome. Sequencing data that were matched perfectly and aligned successfully only once, *i.e.* uniquely aligned sequencing data, were taken out, and the information about the sites of all the uniquely aligned sequencing data was obtained. The number of uniquely aligned sequencing data (recorded as $UR_{sample}$) in each window in each chromosome in the genome corresponding to the alignment result of the sample to be tested was counted, the obtained $UR_{sample}$ and $GC_{ref}$ were plotted to obtain Figure 8A. As shown in Figure 8A, the GC bias introduced via the sequencer caused that more distribution of sequencing data with GC roughly in the [0.35, 0.55] region. The discrete points in Figure 8A were fitted with the smooth spline method into a smooth curve, *i.e.*, Figure 8B, which was a diagram reflecting the relationship between the GC content and the number of sequencing data. In Figure 8B, all the windows were divided at the step length of 1% GC average value. The number of uniquely aligned sequencing data, *i.e.* $M_{fit}$, in a window corresponding to each GC average value could be obtained from fitted data. According to the formula $W_{GC} = M/M_{fit}$, the predetermined weighting coefficient $W_{GC}$ was obtained, and see Figure 8C for the distribution of the weighting coefficient, where M is the number of uniquely aligned sequencing data from the sample to be tested that fall in windows of an equal GC average value.

The number of uniquely aligned sequencing data (recorded as $UR_{bin}$) falling in the windows of each chromosome in the genome for the sample to be tested was corrected using the obtained weighting coefficient, $UR_{fit}$ (corresponding to each window) = $UR_{bin}* W_{GC}$, the median $UR_i$ (i = 1, 2,..., 22) of the corrected number of uniquely aligned sequencing data was calculated for each chromosome, and a box plot, Figure 8D, was drawn and obtained. It could be seen from Figure 8D that the number of uniquely aligned sequencing data corresponding to chromosome 21 in the sample GP9 was obviously higher than those corresponding to other chromosomes, and it could be determined that aneuploidy existed in chromosome 21.

In addition, the t value test was performed on the chromosomes 13, 18 and 21 in which aneuploidy is common. For these three relatively common aneuploids, we performed the t value test on the data volumes of these three chromosomes according to the following formula,

$$T_{i,j} = \frac{\mu_i - \mu_j}{\sqrt{\frac{\sigma_i^2}{n_i} + \frac{\sigma_j^2}{n_j}}} \quad (\text{formula I})$$

Where $\mu$ is UR; (i = 13, 18, 21; j = 1, 2,..., 22) for some chromosome of the sample to be tested; i and j represent chromosomes i, j; $\sigma$ represents the standard deviation of the distribution of UR for some chromosome; and n represents the number of windows in some chromosome.

Chromosome i was compared as mentioned above with chromosomes 1-12 respectively and the average difference (t-value) was obtained according to the following formula,

$$T_i = \frac{1}{12} * \sum_{j=1}^{12} T_{i,j}$$

Chromosomes 1-12 were selected, since the fluctuation of data of chromosomes 1-12 was relatively small, and the chromosomes of our interest (including chr13, chr18 and chr21) were excluded as far as possible. The thresholds set in this method (for autosomes) were: t-value $\leq$ -4 meaning that the corresponding chromosome was at a high risk of trisomy; -4 < t-value $\leq$ -3.5 meaning that the detection result was uncertain, and samples needed to be retaken to be loaded onto the instrument or libraries needed to be reconstructed to be loaded onto the instrument to determine the detection result; and t-value > -3.5 meaning that the detection result was a low risk. It could be seen from Table 2 that among t-values corresponding to chromosomes 13, 18 and 21 of the sample GP9, the t-value for chromosome 21 had already exceeded the criteria for high-risk of 21-trisomy.

Table 2: Results of the t value test

| Sample name | t value for chromosome 13 | t value for chromosome 18 | t value for chromosome 21 | Result |
|---|---|---|---|---|
| GP9 | 0.96287409 | -2.206632741 | -34.59904702 | 21-trisomy |

In the description of the present specification, by the description of the reference terms "an embodiment", "some embodiments", "example", "particular example", or "some examples" and others it is intended that particular features, structures, materials or characteristics described in conjunction with the embodiment or example are contained in at least one embodiment or example of the present invention. In the present specification, the schematic expression of the above-mentioned terms does not necessarily refer to the same embodiment or example.

**Claims**

1. A method for determining whether a genomic abnormality exists in a fetus, comprising the steps of:

a) separating fetal nucleated red blood cells from a sample obtained from a pregnant woman;

b) amplifying a whole genome of the nucleated red blood cells to obtain an amplified whole genome, constructing a whole-genome sequencing library using the amplified whole genome, and sequencing the whole-genome sequencing library, so as to obtain a sequencing result containing a plurality of sequencing data;

c) determining whether the genomic abnormality exists in the fetus by determining the genomic abnormality in the nucleated red blood cells based on the sequencing result,

wherein the genomic abnormality is chromosomal aneuploidy, and the genomic abnormality in the nucleated red blood cells is determined by follows:

c1) dividing the known sequence of a first chromosome into a plurality of windows, the plurality of windows independently having a predetermined length, respectively;

c2) aligning the sequencing data in the sequencing result with the known sequence of the first chromosome, so as to obtain the number of sequencing data falling in each window;

c3) determining a first parameter based on the number of sequencing data falling in each of the window;

c4) determining whether the nucleated red blood cells have aneuploidy for the first chromosome based on the first parameter,

wherein the first parameter is determined by:

c4-i) setting a predetermined weighting coefficient for the number of sequencing data falling in each window, respectively; and

c4-ii) according to the weighting coefficient, performing weighted averaging on the number of sequencing data falling in each of the window to obtain median of the first chromosome, the median of the first chromosome forming the first parameter of the first chromosome,

wherein the predetermined weighting coefficient is obtained by associating the number of sequencing data falling in each window with the GC content of the respective window,

c5) performing the same treatment on a second chromosome as on the first chromosome, so as to obtain median of the second chromosome, wherein

the first chromosome is at least one selected from the group consisting of human chromosome 21, chromosome 18, chromosome 13, X chromosome and Y chromosome; and

the second chromosome is any one selected from the group consisting of chromosomes 1-12 in the human genome;

c6) performing t value test on the median of the first chromosome and the median of the second chromosome, so as to obtain a difference between the first chromosome and the second chromosome; and

c7) comparing the difference with a predetermined first threshold and a second threshold, and if the difference is lower than the predetermined thresholds, then concluding that the nucleated red blood cells have aneuploidy for the first chromosome, and if the difference is higher than the predetermined thresholds, then concluding that the nucleated red blood cells do not have aneuploidy for the first chromosome.

2. The method according to claim 1, wherein the sample from the pregnant woman is peripheral blood of the pregnant woman, and the gestational age of the pregnant woman is 12-20 weeks,

preferably the step of separating the fetal nucleated red blood cells from the peripheral blood of the pregnant woman further comprises:

performing gradient centrifugation on the peripheral blood using a density gradient reagent, so as to obtain monocytes; and

enriching the nucleated red blood cells from the monocytes using magnetic beads carrying an antibody, wherein the antibody specifically recognizes an antigen on the surface of the nucleated red blood cells,

preferably the antibody is an antibody specifically recognizing CD71.

3. The method according to claim 1, wherein the step of constructing the whole-genome sequencing library using the amplified whole genome further comprises:

fragmenting the amplified whole genome, so as to obtain DNA fragments;

performing end repair on the DNA fragments, so as to obtain end-repaired DNA fragments;

adding bases A to 3' ends of the end-repaired DNA fragments, so as to obtain DNA fragments with a sticky end A; ligating the DNA fragments with the sticky end A to an adapter, so as to obtain a ligation product; performing PCR amplification on the ligation product, so as to obtain a second amplification product; and purifying and recovering the second amplification product, so as to obtain a recovered product, and the recovered product forming the whole-genome sequencing library.

4. The method according to claim 1, wherein the predetermined lengths of the plurality of windows are the same, preferably, the predetermined lengths of the plurality of windows are all 60 kB.

5. The method according to claim 1, wherein the sequencing data falling in each of the windows are uniquely aligned sequencing data.

6. The method according to claim 1, wherein the following formula is used to perform the t value test on the median of the first chromosome and the median of the second chromosome,

$$T_{i,j} = \frac{\mu_i - \mu_j}{\sqrt{\dfrac{\sigma_i{}^2}{n_i} + \dfrac{\sigma_j{}^2}{n_j}}}$$

where $T_{i,j}$ represents the difference between the first chromosome and the second chromosome, $\mu_i$ represents the median of the first chromosome, $\mu_j$ represents the median of the second chromosome, $\sigma_i$ represents the standard deviation of the distribution of the number of sequencing data in each window in the first chromosome, $\sigma_j$ represents the standard deviation of the distribution of the number of sequencing data in each window in the second chromosome, $n_i$ represents the number of the windows in the first chromosome, and $n_j$ represents the number of the windows in the second chromosome.

7. The method according to claim 1, wherein the predetermined first threshold is -4 or less, and the second threshold is -3.5 or greater.

8. The method according to claim 1, wherein the genomic abnormality is a mutation in a predetermined region, and the genomic abnormality exists in the nucleated red blood cells based on the sequencing result is determined by a method comprising:

determining the nucleic acid sequence of the predetermined region in said nucleated red blood cells based on the sequencing result;
aligning the nucleic acid sequence of the predetermined region in the nucleated red blood cells to a control nucleic acid sequence, preferably, the control nucleic acid sequence being a normal human genomic sequence; and
determining whether an abnormality exists in the predetermined region in the nucleated red blood cells based on a result of the alignment.

9. A system for determining whether a genomic abnormality exists, comprising:

a nucleated red blood cell separation device, the nucleated red blood cell separation device being used for separating fetal nucleated red blood cells from a sample obtained from a pregnant woman;
a sequencing device, the sequencing device being used for amplifying a whole genome of the nucleated red blood cells to obtain an amplified whole genome, constructing a whole-genome sequencing library using the amplified whole genome, and sequencing the whole-genome sequencing library, so as to obtain a sequencing result containing a plurality of sequencing data; and
a sequencing result analysis device, the sequencing result analysis device being connected to the sequencing device, so as to receive the sequencing result from the sequencing device, and determining whether a genomic abnormality exists in said nucleated red blood cells based on the sequencing result,
wherein the genomic abnormality is chromosomal aneuploidy, and the sequencing result analysis device being suitable for the following operations:

c1) dividing the known sequence of a first chromosome into a plurality of windows, the plurality of windows independently having a predetermined length, respectively;

c2) aligning the sequencing data in the sequencing result with the known sequence of the first chromosome, so as to obtain the number of sequencing data falling in each window;

c3) determining a first parameter based on the number of sequencing data falling in each of the window; and

c4) determining whether the nucleated red blood cells have aneuploidy for the first chromosome based on the first parameter,

wherein the first parameter is determined by:

c4-i) setting a predetermined weighting coefficient for the number of sequencing data falling in each window, respectively; and

c4-ii) according to the weighting coefficient, performing weighted averaging on the number of sequencing data falling in each of the window to obtain median of the first chromosome, the median of the first chromosome forming the first parameter of the first chromosome,

wherein the predetermined weighting coefficient is obtained by associating the number of sequencing data falling in each window with the GC content of the respective window,

c5) performing the same treatment on a second chromosome as on the first chromosome, so as to obtain median of the second chromosome, wherein

the first chromosome is at least one selected from the group consisting of human chromosome 21, chromosome 18, chromosome 13, X chromosome and Y chromosome; and

the second chromosome is any one selected from the group consisting of chromosomes 1-12 in the human genome;

c6) performing t value test on the median of the first chromosome and the median of the second chromosome, so as to obtain a difference between the first chromosome and the second chromosome; and

c7) comparing the difference with a predetermined first threshold and a second threshold, and if the difference is lower than the predetermined thresholds, then concluding that the nucleated red blood cells have aneuploidy for the first chromosome, and if the difference is higher than the predetermined thresholds, then concluding that the nucleated red blood cells do not have aneuploidy for the first chromosome,

wherein the nucleated red blood cell separation device further comprises:

a monocyte separation unit, the monocyte separation unit being suitable for performing gradient centrifugation on the sample from a pregnant woman using a density gradient reagent, so as to obtain monocytes, wherein the sample from the pregnant woman is preferably peripheral blood of the pregnant woman; and

a magnetic enrichment unit, the magnetic enrichment unit being connected to the monocyte separation unit, and being suitable for enriching nucleated red blood cells from the monocytes using magnetic beads carrying an antibody, wherein the antibody specifically recognizes an antigen on the surface of the nucleated red blood cells.

10. The system according to claim 9, further comprising a whole-genome sequencing library preparation device, the whole-genome sequencing library device being connected to the sequencing device, and providing a whole-genome sequencing library used for sequencing for the sequencing device,

wherein the whole-genome sequencing library preparation device further comprises:

a nucleated red blood cell lysis unit, the nucleated red blood cell lysis unit being connected to the nucleated red blood cell separation device, and receiving and lysing nucleated red blood cells, so as to release the whole genome of the nucleated red blood cells;

a whole genome amplification unit, the whole genome amplification unit being connected to the nucleated red blood cell lysis unit, and used for amplifying the whole genome of the nucleated red blood cells, so as to obtain an amplified whole genome; and

a sequencing library construction unit, the sequencing library construction unit being used for receiving the amplified whole genome, and constructing the whole-genome sequencing library using the amplified whole genome.

11. The system according to claim 9, wherein the sequencing result analysis device further comprises:

a nucleic acid sequence determination unit, the nucleic acid sequence determination unit on a predetermined region being suitable for on the basis of the sequencing result, determining the nucleic acid sequence of the predetermined region in the nucleated red blood cells;

an alignment unit, the alignment unit being connected to the nucleic acid sequence determination unit, and being suitable for aligning the nucleic acid sequence of the predetermined region in the nucleated red blood cells to a control nucleic acid sequence; and

an abnormality determination unit, the abnormality determination unit being connected to the alignment unit, and being suitable for determining whether an abnormality exists in the predetermined region in the nucleated red blood cells based on a result of the alignment.

**Patentansprüche**

1. Eine Methode, um zu bestimmen, ob eine genetische Anomalie in einem Fötus existiert, folgende Schritte umfassend:

a) Abtrennen fötaler kernhaltiger roter Blutkörperchen von einer Probe, die von einer schwangeren Frau erhalten wurde;

b) Vervielfältigen eines kompletten Genoms der kernhaltigen roten Blutkörperchen, um ein vervielfältigtes Gesamtgenom zu erhalten, Erstellen einer Sequenzierbibliothek des Gesamtgenoms unter Verwendung des vervielfältigten Gesamtgenoms, und Sequenzieren der Sequenzierbibliothek des Gesamtgenoms, um ein Sequenzierungsergebnis zu erhalten, das eine Vielzahl von Sequenzierungsdaten enthält;

c) Ermitteln, ob im Fötus die genetische Anomalie existiert durch Ermitteln der genetischen Anomalie in den kernhaltigen roten Blutkörperchen basierend auf dem Sequenzierungsergebnis, wobei die genetische Anomalie eine chromosomale Aneuploidie ist, und die genetische Anomalie in den kernhaltigen roten Blutkörperchen wie folgt ermittelt wird:

c1) Aufteilen der bekannten Sequenz eines ersten Chromosoms in eine Vielzahl von Fenstern, die Vielzahl von Fenstern haben unabhängig voneinander jeweils eine vorbestimmte Größe;

c2) Abgleichen der Sequenzierungsdaten des Sequenzierungsergebnisses mit der bekannten Sequenz des ersten Chromosoms, um die Anzahl der Sequenzierungsdaten, die in jedes Fenster fallen, zu erhalten;

c3) Ermitteln eines ersten Parameters basierend auf der Anzahl der Sequenzierungsdaten, die in jedes Fenster fallen;

c4) Ermitteln ob die kernhaltigen roten Blutkörperchen eine Aneuploidie des ersten Chromosoms aufweisen, basierend auf dem ersten Parameter,

wobei der erste Parameter ermittelt wird durch:

c4-i) Festlegen eines vorbestimmten Gewichtungskoeffizienten für die jeweilige Anzahl an Sequenzierungsdaten, die in jedes Fenster fallen; und

c4-ii) entsprechend dem Gewichtungskoeffizienten Durchführen einer gewichteten Mittelwertbildung über die Anzahl an Sequenzierungsdaten, die in jedes der Fenster fallen, um den Median des ersten Chromosoms zu erhalten, der Median des ersten Chromosoms bildet den ersten Parameter für das erste Chromosom,

wobei der vorbestimmte Gewichtungskoeffizient erhalten wird, indem die Anzahl der Sequenzierungsdaten, die in jedes Fenster fallen, mit dem GC-Gehalt des jeweiligen Fensters in Verbindung gebracht wird,

c5) Behandlung des zweiten Chromosoms wie das erste Chromosom, um den Median des zweiten Chromosoms zu erhalten, wobei

das erste Chromosom mindestens eines ist aus der Gruppe bestehend aus dem menschlichen Chromosom 21, Chromosom 18, Chromosom 13, X-Chromosom und Y-Chromosom; und

das zweite Chromosom ist eines aus der Gruppe bestehend aus den Chromosomen 1-12 des menschlichen Genoms;

c6) Durchführen eines t-Tests an dem Median des ersten Chromosoms und dem Median des zweiten Chromosoms, um den Unterschied zwischen dem ersten Chromosom und dem zweiten Chromosom zu erhalten; und

c7) Vergleichen des Unterschieds mit einem vorbestimmten ersten Schwellwert und einem zweiten Schwellwert, und wenn der Unterschied geringer als die vorbestimmten Schwellwerte ist, folgern, dass die kernhaltigen roten Blutkörperchen eine Aneuploidie des ersten Chromosoms aufweisen, und wenn der Unterschied größer als die vorbestimmten Schwellwerte ist, folgern, dass die kernhaltigen roten Blutkörperchen keine Aneuploidie des ersten Chromosoms aufweisen.

2. Die Methode nach Anspruch 1, wobei die Probe von einer schwangeren Frau peripheres Blut der schwangeren Frau ist und das Gestationsalter der schwangeren Frau 12-20 Wochen beträgt,
vorzugsweise umfasst der Schritt des Abtrennens der fötalen kernhaltigen roten Blutkörperchen von dem peripheren Blut der schwangeren Frau weiterhin:

Durchführung einer Gradientenzentrifugation des peripheren Blutes mit einem Dichtegradientenreagens, um Monozyten zu erhalten; und
Anreichern der kernhaltigen roten Blutkörperchen aus den Monozyten mittels Magnetic Beads, die einen Antikörper tragen, wobei der Antikörper spezifisch ein Antigen an der Oberfläche der kernhaltigen roten Blutkörperchen erkennt,

vorzugsweise ist der Antikörper ein Antikörper, der spezifisch CD71 erkennt.

3. Die Methode nach Anspruch 1, wobei der Schritt des Erstellens der Gesamtgenom-Sequenzierbibliothek unter Verwendung des vervielfältigten Gesamtgenoms weiterhin umfasst:

Fragmentieren des vervielfältigten Gesamtgenoms, um DNA-Fragmente zu erhalten;
Durchführung einer Endenreparatur der DNA-Fragmente, um DNA-Fragmente mit reparierten Enden zu erhalten;
Anfügen von Basen A an die 3'-Enden der DNA-Fragmente mit reparierten Enden, um DNA-Fragmente mit einem klebrigen A-Ende zu erhalten;
Ligieren der DNA-Fragmente mit einem klebrigen A-Ende an einen Adapter, um ein Ligationsprodukt zu erhalten;
Durchführen einer PCR-Vervielfältigung des Ligationsproduktes, um ein zweites Vervielfältigungsprodukt zu erhalten; und
Aufreinigen und Gewinnen des zweiten Vervielfältigungsproduktes, um ein gewonnenes Produkt zu erhalten, und das gewonnene Produkt bildet die Gesamtgenom-Sequenzierbibliothek.

4. Die Methode nach Anspruch 1, wobei die vorbestimmten Größen der Vielzahl von Fenstern gleich sind,
vorzugsweise sind die vorbestimmten Größen der Vielzahl von Fenstern alle 60 kB.

5. Die Methode nach Anspruch 1, wobei die in jedes Fenster fallenden Sequenzierungsdaten eindeutig abgeglichene Sequenzierungsdaten sind.

6. Die Methode nach Anspruch 1, wobei die folgende Formel zur Durchführung des t-Tests an dem Median des ersten Chromosoms und dem Median des zweiten Chromosoms verwendet wird,

$$T_{i,j} = \frac{\mu_i - \mu_j}{\sqrt{\frac{\sigma_i^2}{n_i} + \frac{\sigma_j^2}{n_j}}}$$

wobei $T_{i,j}$ der Unterschied zwischen dem ersten Chromosom und dem zweiten Chromosom ist, $\mu_i$ der Median des ersten Chromosoms ist, $\mu_j$ der Median des zweiten Chromosoms ist, $\sigma_i$ die Standardabweichung der Verteilung der Anzahl der Sequenzierungsdaten in jedem Fenster für das erste Chromosom ist, $\sigma_j$ die Standardabweichung der Verteilung der Anzahl der Sequenzierungsdaten in jedem Fenster für das zweite Chromosom ist, $n_i$ die Anzahl der Fenster beim ersten Chromosom ist, und $n_j$ die Anzahl der Fenster beim zweiten Chromosom ist.

7. Die Methode nach Anspruch 1, wobei der vorbestimmte erste Schwellwert -4 oder kleiner ist, und der zweite Schwellwert -3,5 oder größer ist.

8. Die Methode nach Anspruch 1, wobei die genetische Anomalie eine Mutation in einer vorbestimmten Region ist, und die genetische Anomalie in den kernhaltigen roten Blutkörperchen existiert, basierend auf dem Sequenzierungsergebnis, welches durch eine Methode erhalten wurde, die umfasst:

Ermitteln der Nukleinsäuresequenz der vorbestimmten Region der genannten kernhaltigen roten Blutkörperchen basierend auf dem Sequenzierungsergebnis;

Abgleichen der Nukleinsäuresequenz der vorbestimmten Region der kernhaltigen roten Blutkörperchen mit einer Kontroll-Nukleinsäuresequenz, vorzugsweise ist die Kontroll-Nukleinsäuresequenz eine normale menschliche genomische Sequenz; und

Ermitteln, ob eine Anomalie in der vorbestimmten Region der kernhaltigen roten Blutkörperchen existiert basierend auf dem Ergebnis des Alignements.

9. Ein System zur Ermittlung, ob eine genetische Anomalie existiert, umfassend:

Ein Gerät zur Separierung von kernhaltigen roten Blutkörperchen, wobei das Gerät zur Separierung von kernhaltigen roten Blutkörperchen benutzt wird, um fötale kernhaltige rote Blutkörperchen von einer Probe, die von einer schwangeren Frau gewonnen wurde, abzutrennen;

ein Sequenziergerät, wobei das Sequenziergerät benutzt wird, um ein Gesamtgenom aus den kernhaltigen roten Blutkörperchen zu vervielfältigen um ein vervielfältigtes Gesamtgenom zu erhalten, um eine Gesamtgenom-Sequenzierbibliothek aus dem vervielfältigten Gesamtgenom zu erstellen, und um die Gesamtgenom-Sequenzierbibliothek zu sequenzieren, um ein Sequenzierergebnis zu erhalten, das eine Vielzahl von Sequenzierungsdaten enthält; und

ein Sequenzierergebnis-Analysegerät, wobei das Sequenzierergebnis-Analysegerät mit dem Sequenziergerät verbunden ist, um das Sequenzierergebnis vom Sequenziergerät zu erhalten und um basierend auf dem Sequenzierergebnis zu ermitteln, ob eine genetische Anomalie in den genannten kernhaltigen roten Blutkörperchen existiert,

wobei die genetische Anomalie eine chromosomale Aneuploidie ist, und das Sequenzierergebnis-Analysegerät für folgende Operationen geeignet ist:

c1) Aufteilen der bekannten Sequenz eines ersten Chromosoms in eine Vielzahl von Fenstern, wobei die Vielzahl an Fenstern unabhängig voneinander jeweils eine vorbestimmte Größe haben;

c2) Abgleichen der Sequenzierungsdaten des Sequenzierungsergebnisses mit der bekannten Sequenz des ersten Chromosoms, um die Anzahl der Sequenzierungsdaten, die in jedes Fenster fallen, zu erhalten;

c3) Ermitteln eines ersten Parameters basierend auf der Anzahl der Sequenzierungsdaten die in jedes der Fenster fallen; und

c4) Ermitteln, ob die kernhaltigen roten Blutkörperchen eine Aneuploidie des ersten Chromosoms aufweisen basierend auf dem ersten Parameter,

wobei der erste Parameter ermittelt wird durch:

c4-i) Festlegen eines vorbestimmten Gewichtungskoeffizienten für die jeweilige Anzahl an Sequenzierungsdaten die in jedes Fenster fallen; und

c4-ii) entsprechend dem Gewichtungskoeffizienten Durchführen einer gewichteten Mittelwertbildung über die Anzahl an Sequenzierungsdaten, die in jedes der Fenster fallen, um den Median des ersten Chromosoms zu erhalten, der Median des ersten Chromosoms bildet den ersten Parameter für das erste Chromosom,

wobei der vorbestimmte Gewichtungskoeffizient erhalten wird, indem die Anzahl der Sequenzierungsdaten, die in jedes Fenster fallen, mit dem GC-Gehalt des jeweiligen Fensters in Verbindung gebracht wird,

c5) Behandlung des zweiten Chromosoms wie das erste Chromosom, um den Median des zweiten Chromosoms zu erhalten, wobei

das erste Chromosom mindestens eines ist aus der Gruppe bestehend aus dem menschlichen Chromosom 21, Chromosom 18, Chromosom 13, X-Chromosom und Y-Chromosom; und

das zweite Chromosom ist eines aus der Gruppe bestehend aus den Chromosomen 1-12 des menschlichen Genoms;

c6) Durchführen eines t-Tests an dem Median des ersten Chromosoms und dem Median des zweiten Chromosoms, um den Unterschied zwischen dem ersten Chromosom und dem zweiten Chromosom zu ermitteln; und

c7) Vergleichen des Unterschieds mit einem vorbestimmten ersten Schwellwert und einem zweiten Schwellwert, und wenn der Unterschied geringer als die vorbestimmten Schwellwerte ist, folgern, dass die kernhaltigen roten Blutkörperchen eine Aneuploidie des ersten Chromosoms aufweisen, und wenn der Unterschied größer als die vorbestimmten Schwellwerte ist, folgern, dass die kernhaltigen roten Blutkörperchen keine Aneuploidie des ersten Chromosoms aufweisen,
wobei das Gerät zur Separierung von kernhaltigen roten Blutkörperchen weiterhin umfasst:

Ein Gerät zur Separierung von Monozyten, wobei das Gerät zur Separierung von Monozyten dazu geeignet ist, eine Gradientenzentrifugation der Probe einer schwangeren Frau mit einem Dichtegradientenreagens durchzuführen, um Monozyten zu erhalten, wobei die Probe einer schwangeren Frau vorzugsweise peripheres Blut der schwangeren Frau ist; und
ein Gerät zur magnetischen Anreicherung, wobei das Gerät zur magnetischen Anreicherung mit dem Gerät zur Separierung von Monozyten verbunden ist und dazu geeignet ist, kernhaltige rote Blutkörperchen aus den Monozyten mittels Magnetic Beads, die einen Antikörper tragen, anzureichern, wobei der Antikörper spezifisch ein Antigen an der Oberfläche der kernhaltigen roten Blutkörperchen erkennt.

**10.** Das System nach Anspruch 9, weiterhin umfassend ein Gerät zur Herstellung einer Gesamtgenom-Sequenzierbibliothek, wobei das Gerät zur Herstellung einer Gesamtgenom-Sequenzierbibliothek mit dem Sequenziergerät verbunden ist, und eine Gesamtgenom-Sequenzierbibliothek bereitstellt, welche von dem Sequenziergerät zur Sequenzierung verwendet wird,
wobei das Gerät zur Herstellung einer Gesamtgenom-Sequenzierbibliothek weiterhin umfasst:

ein Gerät zur Lyse von kernhaltigen roten Blutkörperchen, wobei das Gerät zur Lyse von kernhaltigen roten Blutkörperchen mit dem Gerät zur Separierung von kernhaltigen roten Blutkörperchen verbunden ist und kernhaltige rote Blutkörperchen erhält und lysiert, um das Gesamtgenom der kernhaltigen roten Blutkörperchen freizusetzen;
ein Gerät zur Vervielfältigung eines Gesamtgenoms, wobei das Gerät zur Vervielfältigung eines Gesamtgenoms mit dem Gerät zur Lyse von kernhaltigen roten Blutkörperchen verbunden ist und zur Vervielfältigung des Gesamtgenoms der kernhaltigen roten Blutkörperchen verwendet wird, um ein vervielfältigtes Gesamtgenom zu erhalten; und
ein Gerät zur Herstellung einer Sequenzierbibliothek, wobei das Gerät zur Herstellung einer Sequenzierbibliothek dazu verwendet wird, das vervielfältigte Gesamtgenom zu erhalten und eine Gesamtgenom-Sequenzierbibliothek aus dem vervielfältigten Gesamtgenom herzustellen.

**11.** Das System nach Anspruch 9, wobei das Sequenzierergebnis-Analysegerät weiterhin umfasst:

Ein Gerät zur Ermittlung einer Nukleinsäuresequenz, wobei das Gerät zur Ermittlung einer Nukleinsäuresequenz dazu geeignet ist, basierend auf dem Sequenzierergebnis für eine vorbestimmte Region die Nukleinsäuresequenz der vorbestimmten Region der kernhaltigen roten Blutkörperchen zu ermitteln;
ein Gerät zum Abgleichen, wobei das Gerät zum Abgleichen mit dem Gerät zur Ermittlung einer Nukleinsäuresequenz verbunden ist und zum Abgleichen der Nukleinsäuresequenz der vorbestimmten Region der kernhaltigen roten Blutkörperchen mit einer Kontroll-Nukleinsäuresequenz geeignet ist; und
ein Gerät zum Ermitteln einer Anomalie, wobei das Gerät zum Ermitteln einer Anomalie mit dem Gerät zum Abgleichen verbunden ist und dazu geeignet ist, basierend auf dem Ergebnis des Abgleichens zu ermitteln, ob in der vorbestimmten Region der kernhaltigen roten Blutkörperchen eine Anomalie existiert.

**Revendications**

**1.** Procédé pour déterminer si une anomalie génomique existe dans un foetus, comprenant les étapes de :

a) séparation de globules rouges nucléés foetaux d'un échantillon obtenu auprès d'une femme enceinte ;
b) amplification d'un génome entier des globules rouges nucléés pour obtenir un génome entier amplifié, construction d'une banque de séquençage de génome entier à l'aide du génome entier amplifié, et séquençage de la banque de séquençage de génome entier, de manière à obtenir un résultat de séquençage contenant une pluralité de données de séquençage ;
c) fait de déterminer si l'anomalie génomique existe dans le foetus en déterminant l'anomalie génomique dans les globules rouges nucléés d'après le résultat de séquençage,
dans lequel l'anomalie génomique est une aneuploïdie chromosomique, et l'anomalie génomique dans les globules rouges nucléés et déterminée par les éléments suivants :

c1) division de la séquence connue d'un premier chromosome en une pluralité de fenêtres, la pluralité de fenêtres ayant indépendamment une longueur prédéterminée, respectivement ;

c2) alignement des données de séquençage dans le résultat de séquençage avec la séquence connue du premier chromosome, de manière à obtenir le nombre de données de séquençage entrant dans chaque fenêtre ;

c3) détermination d'un premier paramètre d'après le nombre de données de séquençage entrant dans chacune des fenêtres ;

c4) fait de déterminer si les globules rouges nucléés ont une aneuploïdie pour le premier chromosome d'après le premier paramètre,

dans lequel le premier paramètre est déterminé par :

c4-i) établissement d'un coefficient de pondération prédéterminé pour le nombre de données de séquençage entrant dans chaque fenêtre, respectivement ; et

c4-ii) selon le coefficient de pondération, réalisation d'un moyennage pondéré sur le nombre de données de séquençage entrant dans chacune des fenêtres de manière à obtenir la médiane du premier chromosome, la médiane du premier chromosome formant le premier paramètre du premier chromosome,

dans lequel le coefficient de pondération prédéterminé est obtenu en associant le nombre de données de séquençage entrant dans chaque fenêtre avec la teneur en GC de la fenêtre respective,

c5) réalisation sur un second chromosome du même traitement que sur le premier chromosome, de manière à obtenir une médiane du second chromosome, dans lequel

le premier chromosome est au moins un élément choisi dans le groupe consistant en le chromosome 21, le chromosome 18, le chromosome 13, le chromosome X et le chromosome Y humains ; et

le second chromosome est l'un quelconque des éléments choisis dans le groupe consistant en les chromosomes 1 à 12 dans le génome humain ;

c6) réalisation d'un test de valeur t sur la médiane du premier chromosome et la médiane du second chromosome, de manière à obtenir une différence entre le premier chromosome et le second chromosome ; et

c7) comparaison de la différence avec un premier seuil et un second seuil prédéterminés, et si la différence est plus faible que les seuils prédéterminés, alors le fait de conclure que les globules rouges nucléés ont une aneuploïdie pour le premier chromosome, et si la différence est plus élevée que les seuils prédéterminés, alors le fait de conclure que les globules rouges nucléés n'ont pas d'aneuploïdie pour le premier chromosome.

2. Procédé selon la revendication 1, dans lequel l'échantillon provenant de la femme enceinte et du sang périphérique de la femme enceinte, et l'âge gestationnel de la femme enceinte est de 12 à 20 semaines, de préférence l'étape de séparation des globules rouges nucléés foetaux du sang périphérique de la femme enceinte comprend en outre :

la réalisation d'une centrifugation par gradient sur le sang périphérique à l'aide d'un réactif à gradient de densité, de manière à obtenir des monocytes ; et

l'enrichissement des globules rouges nucléés provenant des monocytes à l'aide de billes magnétiques portant un anticorps, dans lequel l'anticorps reconnaît spécifiquement un antigène sur la surface des globules rouges nucléés,

de préférence l'anticorps est un anticorps reconnaissant spécifiquement CD71.

3. Procédé selon la revendication 1, dans lequel l'étape de construction de la banque de séquençage de génome entier à l'aide du génome entier amplifié comprend en outre :

la fragmentation du génome entier amplifié, de manière à obtenir des fragments d'ADN ;

la réalisation d'une réparation d'extrémité sur les fragments d'ADN, de manière à obtenir les fragments d'ADN à extrémités réparées ;

l'ajout de bases A à des extrémités 3' des fragments d'ADN à extrémités réparées, de manière à obtenir des fragments d'ADN avec une extrémité cohésive A;

la ligature des fragments d'ADN avec l'extrémité cohésive A à un adaptateur, de manière à obtenir un produit de ligature ;

la réalisation d'une amplification par PCR sur le produit de ligature, de manière à obtenir un second produit d'amplification ; et

la purification et la récupération du second produit d'amplification, de manière à obtenir un produit récupéré, et le produit récupéré formant la banque de séquençage de génome entier.

4. Procédé selon la revendication 1, dans lequel les longueurs prédéterminées de la pluralité de fenêtres sont les mêmes,

de préférence, les longueurs prédéterminées de la pluralité de fenêtres sont toutes de 60 kB.

5. Procédé selon la revendication 1, dans lequel les données de séquençage entrant dans chacune des fenêtres sont des données de séquençage alignées de manière unique.

6. Procédé selon la revendication 1, dans lequel la formule suivante est utilisée pour réaliser le test de valeur t sur la médiane du premier chromosome et la médiane du second chromosome,

$$T_{i,j} = \frac{\mu_i - \mu_j}{\sqrt{\dfrac{\sigma_i{}^2}{n_i} + \dfrac{\sigma_j{}^2}{n_j}}}$$

où $T_{i,j}$ représente la différence entre le premier chromosome et le second chromosome, $\mu_i$ représente la médiane du premier chromosome, $\mu_j$ représente la médiane du second chromosome, $\sigma_i$ représente l'écart-type de la distribution du nombre de données de séquençage dans chaque fenêtre dans le premier chromosome, $\sigma_j$ représente l'écart-type de la distribution du nombre de données de séquençage dans chaque fenêtre dans le second chromosome, $n_i$ représente le nombre de fenêtres dans le premier chromosome, et $n_j$ représente le nombre de fenêtres dans le second chromosome.

7. Procédé selon la revendication 1, dans lequel le premier seuil prédéterminé est de -4 ou moins, et le second seuil est de -3,5 ou plus.

8. Procédé selon la revendication 1, dans lequel l'anomalie génomique est une mutation dans une région prédéterminée, et l'anomalie génomique existant dans les globules rouges nucléés d'après le résultat de séquençage est déterminée par un procédé comprenant :

la détermination de la séquence d'acide nucléique de la région prédéterminée dans lesdits globules rouges nucléés d'après le résultat de séquençage ;

l'alignement de la séquence d'acide nucléique de la région prédéterminée dans les globules rouges nucléés avec une séquence d'acide nucléique témoin, de préférence la séquence d'acide nucléique témoin est une séquence génomique humaine normale ; et

le fait de déterminer si une anomalie existe dans la région prédéterminée dans les globules rouges nucléés d'après un résultat de l'alignement.

9. Système pour déterminer si une anomalie génomique existe, comprenant :

un dispositif de séparation de globules rouges nucléés, le dispositif de séparation de globules rouges nucléés étant utilisé pour séparer des globules rouges nucléés foetaux d'un échantillon obtenu auprès d'une femme enceinte ;

un dispositif de séquençage, le dispositif de séquençage étant utilisé pour amplifier un génome entier des globules rouges nucléés pour obtenir un génome entier amplifié, construire une banque de séquençage de génome entier à l'aide du génome entier amplifié, et séquencer la banque de séquençage de génome entier, de manière à obtenir un résultat de séquençage contenant une pluralité de données de séquençage; et

un dispositif d'analyse de résultat de séquençage, le dispositif d'analyse de résultat de séquençage étant connecté au dispositif de séquençage, de manière à recevoir le résultat de séquençage du dispositif de séquençage, et déterminer si une anomalie génomique existe dans lesdits globules rouges nucléés d'après le résultat de séquençage, dans lequel l'anomalie génomique est une aneuploïdie chromosomique, et le dispositif d'analyse de résultat de séquençage étant approprié pour les opérations suivantes :

c1) division de la séquence connue d'un premier chromosome en une pluralité de fenêtres, la pluralité de fenêtres ayant indépendamment une longueur prédéterminée, respectivement ;

c2) alignement des données de séquençage dans le résultat de séquençage avec la séquence connue du premier chromosome, de manière à obtenir le nombre de données de séquençage entrant dans chaque fenêtre ;

c3) détermination d'un premier paramètre d'après le nombre de données de séquençage entrant dans chacune des fenêtres ; et

c4) fait de déterminer si les globules rouges nucléés ont une aneuploïdie pour le premier chromosome d'après le premier paramètre,

dans lequel le premier paramètre est déterminé par :

c4-i) établissement d'un coefficient de pondération prédéterminé pour le nombre de données de séquençage entrant dans chaque fenêtre, respectivement ; et

c4-ii) selon le coefficient de pondération, réalisation d'un moyennage pondéré sur le nombre de données de séquençage entrant dans chacune des fenêtres de manière à obtenir la médiane du premier chromosome, la médiane du premier chromosome formant le premier paramètre du premier chromosome,

dans lequel le coefficient de pondération prédéterminé est obtenu en associant le nombre de données de séquençage entrant dans chaque fenêtre avec la teneur en GC de la fenêtre respective,

c5) réalisation sur un second chromosome du même traitement que sur le premier chromosome, de manière à obtenir une médiane du second chromosome, dans lequel

le premier chromosome est au moins un élément choisi dans le groupe consistant en le chromosome 21, le chromosome 18, le chromosome 13, le chromosome X et le chromosome Y humains ; et

le second chromosome est l'un quelconque des éléments choisis dans le groupe consistant en les chromosomes 1 à 12 dans le génome humain ;

c6) réalisation d'un test de valeur t sur la médiane du premier chromosome et la médiane du second chromosome, de manière à obtenir une différence entre le premier chromosome et le second chromosome ; et

c7) comparaison de la différence avec un premier seuil et un second seuil prédéterminés, et si la différence est plus faible que les seuils prédéterminés, alors de fait de conclure que les globules rouges nucléés ont une aneuploïdie pour le premier chromosome, et si la différence est plus élevée que les seuils prédéterminés, alors le fait de conclure que les globules rouges nucléés n'ont pas d'aneuploïdie pour le premier chromosome,

dans lequel le dispositif de séparation de globules rouges nucléés comprend en outre :

une unité de séparation de monocytes, l'unité de séparation de monocytes étant appropriée pour réaliser une centrifugation par gradient sur l'échantillon provenant d'une femme enceinte à l'aide d'un réactif à gradient de densité, de manière à obtenir des monocytes, dans lequel l'échantillon provenant de la femme enceinte est de préférence du sang périphérique de la femme enceinte ; et

une unité d'enrichissement magnétique, l'unité d'enrichissement magnétique étant connectée à l'unité de séparation de monocytes, et étant appropriée pour enrichir des globules rouges nucléés provenant des monocytes à l'aide de billes magnétiques portant un anticorps, dans lequel l'anticorps reconnaît spécifiquement un antigène sur la surface des globules rouges nucléés.

**10.** Système selon la revendication 9, comprenant en outre un dispositif de préparation de banque de séquençage de génome entier, le dispositif de banque de séquençage de génome entier étant connecté au dispositif de séquençage, et fournissant une banque de séquençage de génome entier utilisée pour le séquençage pour le dispositif de séquençage,

dans lequel le dispositif de préparation de banque de séquençage de génome entier comprend en outre :

une unité de lyse de globules rouges nucléés, l'unité de lyse de globules rouges nucléés étant connectée au dispositif de séparation de globules rouges nucléés, et recevant et lysant des globules rouges nucléés, de manière à libérer le génome entier des globules rouges nucléés ;

une unité d'amplification de génome entier, l'unité d'amplification de génome entier étant connectée à l'unité de lyse de globules rouges nucléés, et utilisée pour amplifier le génome entier des globules rouges nucléés, de manière à obtenir un génome entier amplifié ; et

une unité de construction de banque de séquençage, l'unité de construction de banque de séquençage étant utilisée pour recevoir le génome entier amplifié, et construire la banque de séquençage de génome entier à l'aide du génome entier amplifié.

11. Système selon la revendication 9, dans lequel le dispositif d'analyse de résultat de séquençage comprend en outre :

une unité de détermination de séquence d'acide nucléique, l'unité de détermination de séquence d'acide nucléique sur une région prédéterminée étant appropriée pour, d'après le résultat de séquençage, déterminer la séquence d'acide nucléique de la région prédéterminée dans les globules rouges nucléés ;
une unité d'alignement, l'unité d'alignement étant connectée à l'unité de détermination de séquence d'acide nucléique, et étant appropriée pour animer la séquence d'acide nucléique de la région prédéterminée dans les globules rouges nucléés avec une séquence d'acide nucléique témoin ; et
une unité de détermination d'anomalie, l'unité de détermination d'anomalie étant connectée à l'unité d'alignement, et étant appropriée pour déterminer si une anomalie existe dans la région prédéterminée dans les globules rouges nucléés d'après un résultat de l'alignement.

Separating nucleated red blood cells — S10

Sequencing the genome — S20

Determining a genomic abnormality — S30

*Fig. 1*

Sequencing the whole genome of nucleated cells — S100

Dividing the known sequence of a first chromosome into windows — S200

Determining the number of sequencing data falling in each window — S300

Determining a first parameter — S400

Determining aneuploidy in the nucleated red blood cells — S500

*Fig. 2*

1000   100   200   300

| Nucleated red blood cell separation device | Sequencing device | Sequencing result analysis device |

*Fig. 3*

100   101                                                              102

| Monocyte separation unit | | Magnetic enrichment unit |

*Fig. 4*

1000
100                        400                      200                   300

| Nucleated red blood cell separation device | Whole-genome sequencing library preparation device | Sequencing device | Sequencing result analysis device |

*Fig. 5*

400
401                        402                      403

| Nucleated red blood cell lysis unit | Whole genome amplification unit | Sequencing library construction unit |

*Fig. 6*

Sample GP9

Overall results for the sample

Peaks found                                    1

| Size of peak value | Mass concentration | Molar concentration |
|---|---|---|
| 434bp | 3.6ng/μL | 12.5nmol/L |

*Fig. 7*

Number of
uniquely
aligned
reads in
every 60 kb
window

GC content

*Fig. 8A*

*Fig. 8B*

*Fig. 8C*

*Fig. 8D*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ERIC Z. CHEN et al.** Noninvasive Prenatal Diagnosis of Fetal Trisomy 18 and Trisomy 13 by Maternal Plasma DNA Sequencing. *PloS ONE,* 06 July 2011, vol. 6 (7), e21791

- **METZKER ML.** Sequencing technologies-the next generation. *Nat Rev Genet.,* January 2010, vol. 11 (1), 31-46
- **RUSK, NICOLE.** Cheap Third-Generation Sequencing. *Nature Methods,* 01 April 2009, vol. 6 (4), 244-245